# EUROPEAN PATENT SPECIFICATION

(11) **EP 3 613 360 B1**
(45) Date of publication and mention of the grant of the patent: **04.11.2026**
(21) Application number: 19192343.2
(22) Date of filing: 19.08.2019
(51) Int. Cl.: A61B 17/072

(54) **POWERED SURGICAL INSTRUMENTS WITH CLUTCHING ARRANGEMENTS TO CONVERT LINEAR DRIVE MOTIONS TO ROTARY DRIVE MOTIONS**
ANGETRIEBENE CHIRURGISCHE INSTRUMENTE MIT KUPPLUNGSANORDNUNGEN ZUR UMWANDLUNG VON LINEAREN ANTRIEBSBEWEGUNGEN IN ROTIERENDE ANTRIEBSBEWEGUNGEN
INSTRUMENTS CHIRURGICAUX MOTORISÉS COMPORTANT DES AGENCEMENTS D'EMBRAYAGE POUR CONVERTIR DES MOUVEMENTS D'ENTRAÎNEMENT LINÉAIRE EN MOUVEMENTS D'ENTRAÎNEMENT ROTATIF

(30) Priority: 20.08.2018 US 201816105097
(43) Date of publication of application: 26.02.2020
(73) Proprietor: Ethicon LLC, Guaynabo 00969 (PR)
(72) Inventor: HARRIS, Jason L., Cincinnati, Ohio 45242 (US); SHELTON IV, Frederick E, Cincinnati, Ohio 45242 (US); BAXTER III, Chester O., Cincinnati, Ohio 45242 (US)
(74) Representative: Carpmaels & Ransford LLP

(56) References cited:
- EP-A1- 3 034 013
- EP-A1- 3 547 324
- EP-A2- 1 813 211
- EP-A2- 3 338 664

## Description

### BACKGROUND

The present invention relates to surgical instruments and, in various arrangements, to surgical stapling and cutting instruments and staple cartridges for use therewith that are designed to staple and cut tissue. EP3338664A2 discloses a shaft assembly which comprises an end effector configurable in an open configuration and a closed configuration.

### BRIEF DESCRIPTION OF THE DRAWINGS

Various features of the embodiments described herein, together with advantages thereof, may be understood in accordance with the following description taken in conjunction with the accompanying drawings as follows:
FIG. 1 is a perspective view of a powered surgical stapling system;
FIG. 2 is an exploded assembly view of a shaft assembly of the powered surgical stapling system of FIG. 1;
FIG. 3 is a cross-sectional view of a portion of the shaft assembly and a surgical end effector of the powered surgical stapling system of FIG. 1, with an anvil of the surgical end effector in an open position;
FIG. 4 is a top view of a portion of the shaft assembly and surgical end effector of FIG. 3 in an unarticulated position;
FIG. 5 is another top view of a portion of the shaft assembly and surgical end effector of FIG. 3 in an articulated position;
FIG. 6 is an exploded assembly view of a handle or housing of the powered surgical stapling system of FIG. 1;
FIG. 7 is a cross-sectional view of the handle of FIG. 6 with portions of the shaft assembly omitted for clarity;
FIG. 8 is an enlarged cross-sectional view of the handle and shaft assembly of FIG. 7;
FIG. 9 is another enlarged cross-sectional view of the handle and shaft assembly of FIG. 7;
FIG. 10 is another side cross-sectional view of the handle and shaft assembly of FIG. 7 in a position that results in the jaws of the surgical end effector being oriented in an open position;
FIG. 11 is another side cross-sectional view of the handle and shaft assembly of FIG. 7 in a position that results in the closure of the end effector jaws;
FIG. 12 is a graphical comparison of closure forces between a surgical instrument embodiment that employs a progressive closure drive system and two previous surgical instruments that employ different closure drive system arrangements;
FIG. 13A is a graphical depiction of a force to fire (FTF) and a force to close (FTC) experienced by a previous surgical instrument embodiment that employs camming surfaces on an anvil thereof as a firing member or knife thereof travels through the anvil from a proximal-most starting position to a distal-most ending position in the anvil (crosshead distance in inches (1 inch = 25,4 mm));
FIG. 13B is a graphical depiction of a force to fire (FTF) and a force to close (FTC), anvil height and spring height experienced by a surgical instrument embodiment that employs a progressive closure drive system;
FIG. 14 is a partial perspective view of a portion of a shaft assembly;
FIG. 15 is a side elevational view of a portion of another powered surgical instrument;
FIG. 16 is a partial perspective view of a portion of the powered surgical instrument of FIG. 15, with portions thereof omitted for clarity;
FIG. 17 is another partial perspective view of a portion of the powered surgical instrument of FIG. 15, with portions thereof omitted for clarity;
FIG. 18 is another partial perspective view of a portion of the powered surgical instrument of FIG. 15, with portions thereof omitted for clarity;
FIG. 19 is a perspective view of a motor switch system of the powered surgical instrument of FIG. 15, with portions thereof omitted for clarity;
FIG. 20 is a perspective view of a proximal nozzle segment or fin segment of a nozzle assembly of the powered surgical instrument of FIG. 15;
FIG. 21 is a partial perspective view of portions of the powered surgical instrument of FIG. 15 with a distal nozzle portion omitted for clarity;
FIG. 22 is a side elevational view of portions of a chassis portion and a proximal nozzle segment of the powered surgical instrument of FIG. 15 with portions of the nozzle assembly thereof omitted for clarity;
FIG. 23 is a graphic depiction of positions of switches of the switch system of FIG. 19 relative to a position of a switch traveler thereof in relation to an articulated position of an end effector of the powered surgical instrument of FIG. 15;
FIG. 24 is another graphic depiction of positions of switches of the switch system of FIG. 19 relative to another position of the switch traveler in relation to an articulated position of the end effector of the powered surgical instrument of FIG. 15;
FIG. 25 is another graphic depiction of positions of switches of the switch system of FIG. 19 relative to another position of the switch traveler in relation to an articulated position of the end effector of the powered surgical instrument of FIG. 15;
FIG. 26 is another graphic depiction of positions of switches of the switch system of FIG. 19 relative to another position of the switch traveler in relation to an articulated position of the end effector of the powered surgical instrument of FIG. 15;
FIG. 27 depicts shapes of portions of three different switch traveler embodiments that may be employed in the switch system of FIG. 19;
FIG. 28 is a graphical comparison of motor speeds for each geometrical switch traveler shape depicted in FIG. 27 to an articulation angle of a surgical end effector coupled thereto;
FIG. 28A illustrates one form of control circuit that may be employed to control an articulation motor of the powered surgical instrument of FIG. 16;
FIG. 29 is a perspective view of another powered surgical stapling system;
FIG. 30 is an exploded assembly view of a shaft assembly of the powered surgical stapling system of FIG. 29;
FIG. 31 is a perspective view of a portion of a shaft assembly of the powered surgical stapling system of FIG. 29 with portions thereof omitted for clarity;
FIG. 32A is a top cross-sectional view of a portion of the shaft assembly of FIG. 31 when a closure member thereof is in a retracted position;
FIG. 32B is another top cross-sectional view of the portion of shaft assembly depicted in FIG. 32 A when the closure member thereof is in an advanced closure position; and
FIG. 33 is a partial cross-sectional perspective view of an articulation lock arrangement.

Corresponding reference characters indicate corresponding parts throughout the several views. The exemplifications set out herein illustrate various embodiments of the invention, in one form, and such exemplifications are not to be construed as limiting the scope of the invention in any manner.

### DETAILED DESCRIPTION

The invention is defined by claim 1.

Numerous specific details are set forth to provide a thorough understanding of the overall structure, function, manufacture, and use of the embodiments as described in the specification and illustrated in the accompanying drawings. Well-known operations, components, and elements have not been described in detail so as not to obscure the embodiments described in the specification. The reader will understand that the embodiments described and illustrated herein are non-limiting examples, and thus it can be appreciated that the specific structural and functional details disclosed herein may be representative and illustrative. Variations and changes thereto may be made without departing from the scope of the claims.

The terms "comprise" (and any form of comprise, such as "comprises" and "comprising"), "have" (and any form of have, such as "has" and "having"), "include" (and any form of include, such as "includes" and "including") and "contain" (and any form of contain, such as "contains" and "containing") are open-ended linking verbs. As a result, a surgical system, device, or apparatus that "comprises," "has," "includes" or "contains" one or more elements possesses those one or more elements, but is not limited to possessing only those one or more elements. Likewise, an element of a system, device, or apparatus that "comprises," "has," "includes" or "contains" one or more features possesses those one or more features, but is not limited to possessing only those one or more features.

The terms "proximal" and "distal" are used herein with reference to a clinician manipulating the handle portion of the surgical instrument. The term "proximal" refers to the portion closest to the clinician and the term "distal" refers to the portion located away from the clinician. It will be further appreciated that, for convenience and clarity, spatial terms such as "vertical", "horizontal", "up", and "down" may be used herein with respect to the drawings. However, surgical instruments are used in many orientations and positions, and these terms are not intended to be limiting and/or absolute.

Various exemplary devices and methods are provided for performing laparoscopic and minimally invasive surgical procedures. However, the reader will readily appreciate that the various methods and devices disclosed herein can be used in numerous surgical procedures and applications including, for example, in connection with open surgical procedures. As the present Detailed Description proceeds, the reader will further appreciate that the various instruments disclosed herein can be inserted into a body in any way, such as through a natural orifice, through an incision or puncture hole formed in tissue, etc. The working portions or end effector portions of the instruments can be inserted directly into a patient's body or can be inserted through an access device that has a working channel through which the end effector and elongated shaft of a surgical instrument can be advanced.

A surgical stapling system can comprise a shaft and an end effector extending from the shaft. The end effector comprises a first jaw and a second jaw. The first jaw comprises a staple cartridge. The staple cartridge is insertable into and removable from the first jaw; however, other embodiments are envisioned in which a staple cartridge is not removable from, or at least readily replaceable from, the first jaw. The second jaw comprises an anvil configured to deform staples ejected from the staple cartridge. The second jaw is pivotable relative to the first jaw about a closure axis; however, other embodiments are envisioned in which the first jaw is pivotable relative to the second jaw. The surgical stapling system further comprises an articulation joint configured to permit the end effector to be rotated, or articulated, relative to the shaft. The end effector is rotatable about an articulation axis extending through the articulation joint. Other embodiments are envisioned which do not include an articulation joint.

The staple cartridge comprises a cartridge body. The cartridge body includes a proximal end, a distal end, and a deck extending between the proximal end and the distal end. In use, the staple cartridge is positioned on a first side of the tissue to be stapled and the anvil is positioned on a second side of the tissue. The anvil is moved toward the staple cartridge to compress and clamp the tissue against the deck. Thereafter, staples removably stored in the cartridge body can be deployed into the tissue. The cartridge body includes staple cavities defined therein wherein staples are removably stored in the staple cavities. The staple cavities are arranged in six longitudinal rows. Three rows of staple cavities are positioned on a first side of a longitudinal slot and three rows of staple cavities are positioned on a second side of the longitudinal slot. Other arrangements of staple cavities and staples may be possible.

The staples are supported by staple drivers in the cartridge body. The drivers are movable between a first, or unfired position, and a second, or fired, position to eject the staples from the staple cavities. The drivers are retained in the cartridge body by a retainer which extends around the bottom of the cartridge body and includes resilient members configured to grip the cartridge body and hold the retainer to the cartridge body. The drivers are movable between their unfired positions and their fired positions by a sled. The sled is movable between a proximal position adjacent the proximal end and a distal position adjacent the distal end. The sled comprises a plurality of ramped surfaces configured to slide under the drivers and lift the drivers, and the staples supported thereon, toward the anvil.

Further to the above, the sled is moved distally by a firing member. The firing member is configured to contact the sled and push the sled toward the distal end. The longitudinal slot defined in the cartridge body is configured to receive the firing member. The anvil also includes a slot configured to receive the firing member. The firing member further comprises a first cam which engages the first jaw and a second cam which engages the second jaw. As the firing member is advanced distally, the first cam and the second cam can control the distance, or tissue gap, between the deck of the staple cartridge and the anvil. The firing member also comprises a knife configured to incise the tissue captured intermediate the staple cartridge and the anvil. It is desirable for the knife to be positioned at least partially proximal to the ramped surfaces such that the staples are ejected ahead of the knife.

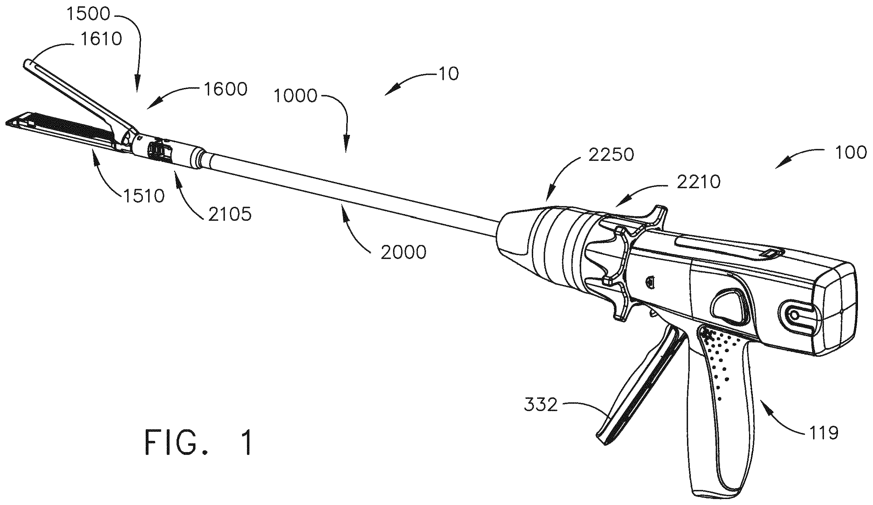

FIG. 1 depicts a motor-driven surgical cutting and fastening instrument 10 that may or may not be reused. In the illustrated embodiment, the instrument 10 includes a housing 100 that comprises a handle 110 that is configured to be grasped, manipulated and actuated by the clinician. In the illustrated example, a dedicated shaft assembly 1000 is operably coupled to the handle 110. In alternative embodiments, however, the handle assembly is configured to be employed with a variety of different interchangeable shaft assemblies that each have a surgical end effector operably coupled thereto that is configured to perform one or more surgical tasks or procedures. For example, the interchangeable shaft assemblies disclosed herein may be employed with various robotic systems, instruments, components and methods disclosed in U.S. Patent Application Serial No. 13/118,241, filed May 27, 2011, now U.S. Patent No. 9,072,535, entitled SURGICAL STAPLING INSTRUMENTS WITH ROTATABLE STAPLE DEPLOYMENT ARRANGEMENTS.

As the present Detailed Description proceeds, it will be understood that the various aspects of the shaft assembly 1000 may also be effectively employed in connection with robotically-controlled surgical systems. Thus, the term "housing" may also encompass a housing or similar portion of a robotic system that houses or otherwise operably supports or is otherwise associated with at least one drive system that is configured to generate and apply at least one control motion which could be used to actuate the interchangeable shaft assemblies disclosed herein and their respective equivalents. The term "frame" may refer to a portion of a handheld surgical instrument. The term "frame" may also represent a portion of a robotically controlled surgical instrument and/or a portion of the robotic system that may be used to operably control a surgical instrument. In addition, various components may be "housed" or contained in the housing or various components may be "associated with" a housing. In such instances, the components may not be contained with the housing or supported directly by the housing.

The illustrated embodiment is an endoscopic instrument and, in general, the embodiments of the instrument 10 described herein are endoscopic surgical cutting and fastening instruments. It should be noted, however, that according to various embodiments, the instrument may be a non-endoscopic surgical cutting and fastening instrument, for example. Various surgical instruments are disclosed in U.S. Patent No. 7,845,537, entitled SURGICAL INSTRUMENT HAVING RECORDING CAPABILITIES; U.S. Patent No. 8,608,045, entitled POWERED SURGICAL CUTTING AND STAPLING APPARATUS WITH MANUALLY RETRACTABLE FIRING SYSTEM; and U.S. Patent No. 9,050,083, entitled MOTORIZED SURGICAL INSTRUMENT.

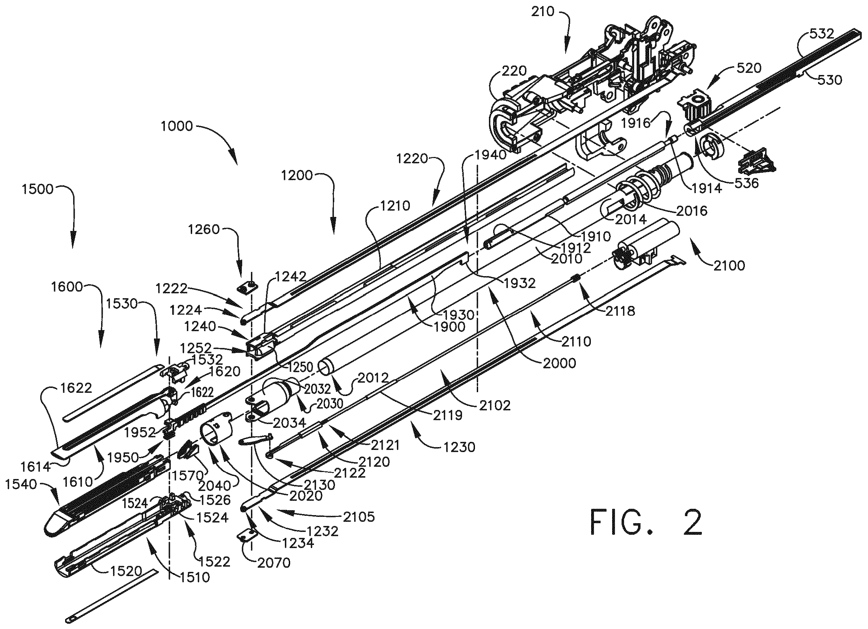

Turning to FIG. 2, in the illustrated example, the shaft assembly 1000 includes an end effector 1500 that is configured to cut and staple tissue. The end effector 1500 comprises a first jaw 1510 and a second jaw 1600 that is movably supported on the first jaw 1510. The first jaw comprises an elongated channel 1520 that is configured to operably support a surgical staple cartridge 1540 therein. The second jaw 1600 comprises an anvil 1610 that includes an elongated anvil body 1612 and an anvil mounting portion 1620. Alternative arrangements are contemplated, however, wherein the first jaw comprises an anvil and the second jaw comprises a surgical staple cartridge or a channel configured to support a surgical staple cartridge. In the illustrated example, the elongated anvil body 1612 includes a staple forming undersurface 1614 thereon that is adapted for confronting relationship with respect to the surgical staple cartridge 1540. The anvil 1610 is pivotally supported on or movably supported on the elongated channel 1520 by a pair of anvil trunnions 1622 that are formed on the anvil mounting portion 1620. Each trunnion 1622 is pivotally received in a corresponding trunnion cradle 1524 formed in a proximal end portion of the elongated channel 1520. The trunnions 1622 are pivotally retained within their respective cradles 1524 by an anvil retainer 1530.

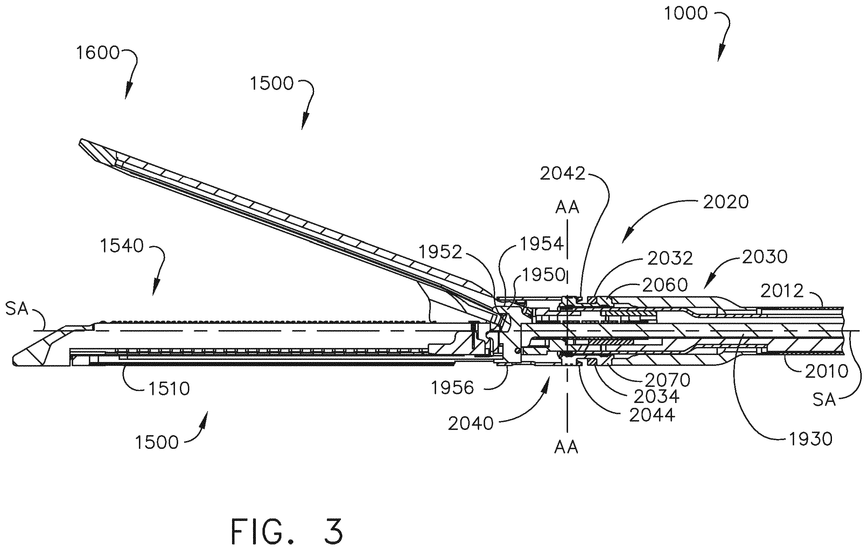

Still referring to FIG. 2, the shaft assembly 1000 includes a spine assembly 1200 that includes a spine shaft 1210 that is configured to, one, slidably support a firing member assembly 1900 therein and, two, slidably support a closure member assembly 2000 which extends around the spine assembly 1200. The spine assembly 1200 further includes upper and lower spine stays 1220 and 1230 that are supported by the spine shaft 1210. As can be seen in FIG. 2, a distal end 1212 of spine shaft 1210 terminates in an upper lug mount feature 1240 and in a lower lug mount feature 1250. The upper lug mount feature 1240 is formed with a lug slot 1242 therein that is adapted to mountingly support the distal end 1222 of the upper spine stay 1220 therein. Similarly, the lower lug mount feature 1250 is formed with a lug slot 1252 therein that is adapted to mountingly support the distal end 1232 of the lower spine stay 1230 therein. The distal end 1222 of the upper spine stay 1220 includes a pivot socket 1224 therein that is adapted to rotatably receive therein a pivot pin 1532 that is formed on a channel cap or anvil retainer 1530. The distal end 1232 of the lower spine stay 1230 includes lower pivot pin 1234 that adapted to be received within a pivot hole (not shown) formed in the proximal end portion 1522 of the elongated channel 1520. The lower pivot pin 1234 is vertically aligned with the pivot socket 1224 to define an articulation axis AA about which the surgical end effector 1500 may articulate relative to the shaft 1000. See FIG. 3.

In the illustrated arrangement, the closure member assembly 2000 comprises a proximal closure tube segment or closure member segment 2010. The proximal closure tube segment 2010 is operably coupled to a double pivot closure sleeve assembly 2020 that defines an articulation joint 2105 about which the end effector 1500 may articulate relative to the remaining portion of the shaft assembly 1000. Other shaft assemblies, however, may not be capable of articulation. As can be seen in FIG. 2, in one form, the double pivot closure sleeve assembly 2020 comprises an intermediate closure tube segment 2030 that is attached to a distal end 2012 of the proximal closure tube segment 2010. In addition, the double pivot closure sleeve assembly 2020 includes an end effector closure tube or distal closure tube 2040 that has upper and lower distally projecting tangs 2042, 2044. An upper double pivot link 2060 includes upwardly projecting distal and proximal pivot pins that engage respectively an upper distal pin hole in the upper proximally projecting tang 2042 and an upper proximal pin hole in an upper distally projecting tang 2032 on the intermediate closure tube segment 2030. A lower double pivot link 2070 includes upwardly projecting distal and proximal pivot pins that engage respectively a lower distal pin hole in the lower proximally projecting tang 2044 and a lower proximal pin hole in the lower distally projecting tang 2034. See FIGS. 2 and 3.

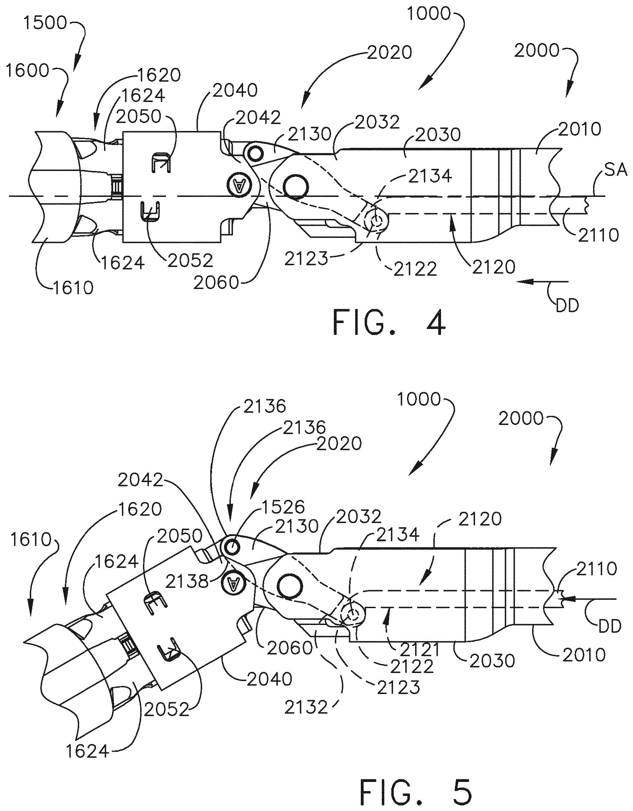

As will be discussed in further detail below, the anvil 1610 is moved from an open position to a closed position by translating the closure member assembly 2000 in the distally (direction "DD"). The anvil 1610 is opened by proximally translating the closure member assembly 2000 which causes the end effector closure sleeve 2020 to interact with the anvil 1610 and pivot it to an open position. Referring to FIGS. 4 and 5, in at least one arrangement, the distal closure member or end effector closure tube 2040 employs two axially offset, proximal and distal positive jaw opening features 2050 and 2052. In FIGS. 4 and 5, the proximal positive jaw opening feature 2050 is located on the right side (as viewed by a user of the tool assembly) of the shaft axis SA. The positive jaw opening features 2050, 2052 are configured to interact with corresponding relieved areas (not shown) and stepped portions (not shown) that are formed on the anvil mounting portion 1620 as described in further detail in U.S. Patent Application Serial No. 15/635,631, filed June 28, 2017, entitled SURGICAL INSTRUMENT WITH AXIALLY MOVABLE CLOSURE MEMBER. Other jaw opening arrangements may also be employed.

In the illustrated example as well as other anvil configurations disclosed in references incorporated herein, the anvil mounting portion 1620 has a cam surface or cam surfaces 1624 formed thereon. As the end effector closure tube 2040 is moved distally, a cam surface formed on a distal end of the end effector closure tube 2040 interacts with the cam surfaces 1624 on the anvil mounting portion 1620 to cam the anvil 1610 into a closed position.

As was also indicated above, the shaft assembly 1000 further includes a firing member assembly 1900 that is supported for axial travel within the spine shaft 1210. The firing member assembly 1900 includes an intermediate firing shaft portion 1910 that is configured for attachment to a distal cutting portion or knife bar 1930. The intermediate firing shaft portion 1910 may include a longitudinal slot 1912 in the distal end thereof which can be configured to receive a tab 1932 on the proximal end of the distal knife bar 1930. The longitudinal slot 1932 and the proximal end tab 1932 can be sized and configured to permit relative movement therebetween and can comprise a slip joint 1940. The slip joint 1940 can permit the knife bar 1930 to move axially relative to the intermediate firing shaft portion 1910 to accommodate articulation of the end effector 1500, for example. The knife bar 1930 includes a knife portion 1950 that includes a blade or tissue cutting edge 1952 and includes an upper anvil engagement tab 1954 and lower channel engagement tabs 1956.

In the illustrated example, the surgical end effector 1500 is selectively articulatable about the articulation axis AA by an articulation drive system 2100. In one form, the articulation drive system 2100 includes proximal articulation driver 2110 that is operably coupled to an intermediate articulation driver 2120 that is pivotally coupled to a distal articulation link 2130. As can be most particularly seen in FIGS. 4 and 5, an offset attachment lug 2122 is formed on a distal end of the intermediate articulation driver 2120. A pivot hole 2123 is formed in the offset attachment lug 2122 and is configured to pivotally receive therein a proximal link pin 2134 formed on the proximal end 2132 of the distal articulation link 2130. A distal end 2136 of the articulation link 2120 includes a pivot hole 2138 that is configured to pivotally receive therein a channel pin 1526 that is formed on the proximal end portion 1522 of the elongated channel 1520. Thus, axial movement of intermediate articulation driver 2120 will thereby apply articulation motions to the elongated channel 1520 to thereby cause the surgical end effector 1500 to articulate about the articulation axis AA relative to the spine assembly 1200.

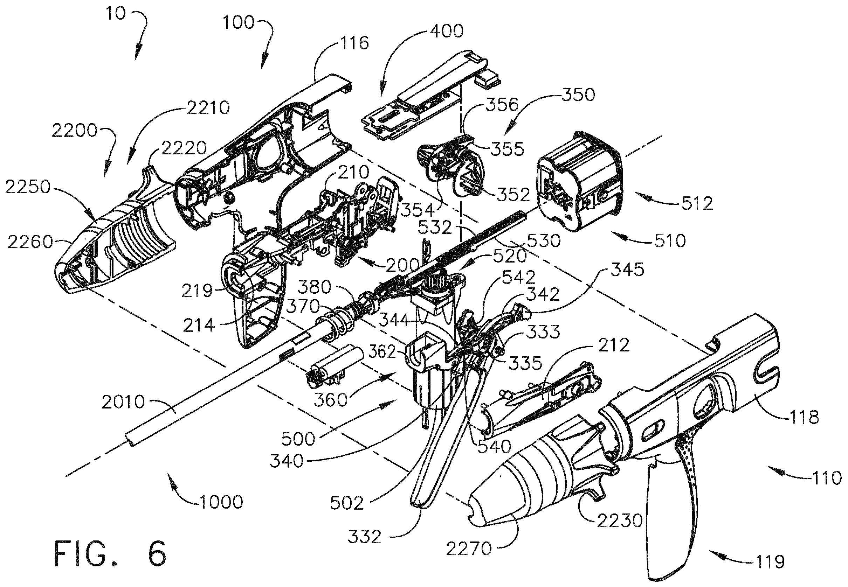

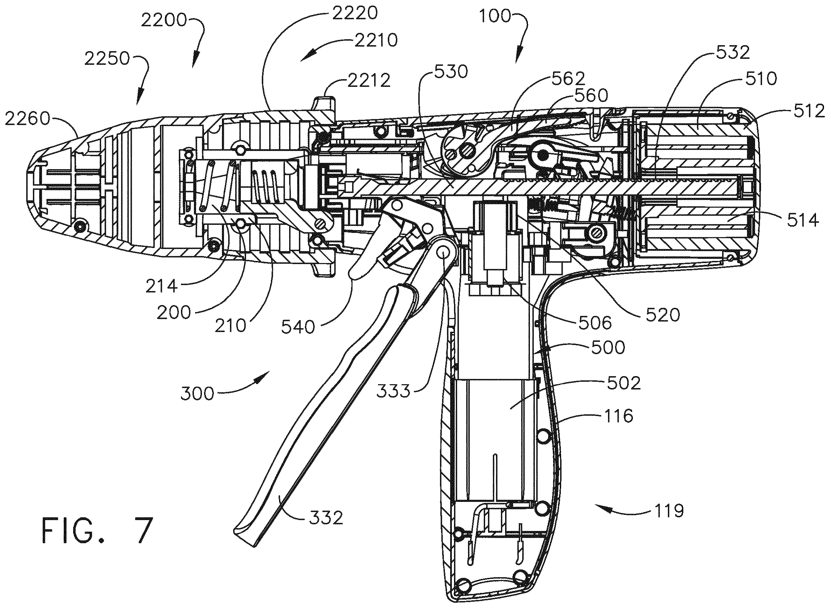

Turning now to FIGS. 6 and 7, the handle 110 comprises handle housing segments 116, 118 cooperate to form a pistol grip portion 119 that can be gripped and manipulated by the clinician. As will be discussed in further detail below, the handle 110 operably supports a plurality of drive systems therein that are configured to generate and apply various control motions to the shaft assembly 1200. Referring now to FIG. 2, the handle 110 may further include a frame assembly or chassis 200 that operably supports a plurality of drive systems. For example, the frame assembly 200 can operably support a "first" or closure drive system, generally designated as 300, which may be employed to apply closing and opening motions to the end effector 1500 of the shaft assembly 1000. In the illustrated example, the frame assembly 200 includes a right frame portion 210 and a frame cap 212 that is attached thereto by snap features, lugs, screws, etc. to define a shuttle cavity 214 therein. See FIGS. 6 and 7.

In at least one form, the closure drive system 300 may include an actuator in the form of a closure trigger 332 that is pivotally supported by the frame assembly 200. More specifically, as illustrated in FIGS. 6 and 7, the closure trigger 332 is pivotally coupled to the frame assembly 200 by a pin 333. Such arrangement enables the closure trigger 332 to be manipulated by a clinician such that when the clinician grips the pistol grip portion 119 of the handle 100, the closure trigger 332 may be easily pivoted from a starting or "unactuated" position to an "actuated" position and more particularly to a fully compressed or fully actuated position. The closure trigger 332 may be biased into the unactuated position by spring or other biasing arrangement. In various forms, the closure drive system 300 further includes a closure linkage assembly 340 that is pivotally coupled to the closure trigger 1032. As can be seen in FIG. 6, the closure linkage assembly 340 may include a first closure link 342 and a second closure link 344 that are each pivotally coupled to the closure trigger 332 by a pin 335.

Still referring to FIG. 6, it can be observed that the first closure link 342 may have a locking wall or end 345 thereon that is configured to cooperate with a closure release assembly 350 that is pivotally coupled to the right frame portion 210. In at least one form, the closure release assembly 350 may comprise a release button assembly 352 that has a distally protruding locking pawl 354 formed thereon. The release button assembly 352 may be pivoted in a counterclockwise direction by a release spring (not shown). As the clinician depresses the closure trigger 332 from its unactuated position towards the pistol grip portion 119 of the handle 100, the first closure link 342 pivots upward to a point wherein the locking pawl 354 drops into retaining engagement with the locking wall 345 on the first closure link 344 thereby preventing the closure trigger 332 from returning to the unactuated position. Thus, the closure release assembly 350 serves to lock the closure trigger 332 in the fully actuated position. When the clinician desires to unlock the closure trigger 332 to permit it to be biased to the unactuated position, the clinician simply pivots the closure release button assembly 352 such that the locking pawl 354 is moved out of engagement with the locking wall 345 on the first closure link 344. When the locking pawl 354 has been moved out of engagement with the first closure link 344, the closure trigger 332 may pivot back to the unactuated position. Other closure trigger locking and release arrangements may also be employed.

In the illustrated example, an arm 355 extends from the closure release button 352. A magnetic element 356, such as a permanent magnet, for example, may be mounted to the arm 355. When the closure release button 352 is rotated from its first position to its second position, the magnetic element 356 can move toward a circuit board 400. The circuit board 400 can include at least one sensor that is configured to detect the movement of the magnetic element 356. In at least one embodiment, for example, a "Hall Effect" sensor (not shown) can be mounted to the bottom surface of the circuit board 400. The Hall Effect sensor can be configured to detect changes in a magnetic field surrounding the Hall Effect sensor that are caused by the movement of the magnetic element 356. The Hall Effect sensor can be in signal communication with a microcontroller, for example, which can determine whether the closure release button 352 is in its first position, which is associated with the unactuated position of the closure trigger 332 and the open configuration of the end effector, its second position, which is associated with the actuated position of the closure trigger 332 and the closed configuration of the end effector, and/or any position between the first position and the second position.

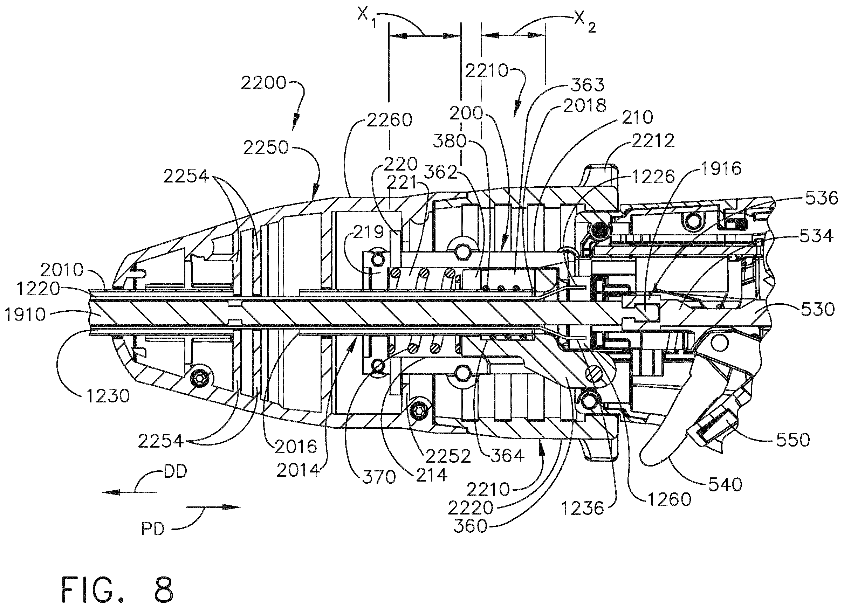

In at least one form, the handle 100 and the frame assembly 200 operably support another drive system referred to herein as a firing drive system 500 that is configured to apply firing motions to the firing member assembly 1900 in the shaft assembly 1000. The firing drive system 500 may also be referred to herein as a "second drive system". The firing drive system 500 may employ an electric motor 502 that is located in the pistol grip portion 119 of the handle 100. In various forms, the motor 502 may be a DC brushed driving motor having a maximum rotation of, approximately, 25,000 RPM, for example. In other arrangements, the motor may include a brushless motor, a cordless motor, a synchronous motor, a stepper motor, or any other suitable electric motor. The motor 502 may be powered by a power source 510 that in one form may comprise a removable power pack 512. As can be seen in FIG. 8, for example, the power pack 512 may support a plurality of batteries 514 therein. Batteries 514 may each comprise, for example, a Lithium Ion ("LI") or other suitable battery. The power pack 512 is configured for removable operable attachment to the circuit board assembly 400 which is also operably coupled to the motor 502. A number of batteries 514 may be connected in series may be used as the power source for the surgical instrument 10. In addition, the power source 510 may be replaceable and/or rechargeable.

As outlined above with respect to other various forms, the electric motor 502 can include a rotatable shaft 506 that operably interfaces with a gear reducer assembly 520 that is mounted in meshing engagement with a with a set, or rack, of drive teeth on a longitudinally-movable drive member 530. An attachment lug 1916 is formed on a proximal end 1914 of the intermediate firing shaft portion 1910. The attachment lug 1916 is configured to be received within an attachment cradle 536 that is formed in a distal end of the longitudinally movable drive member 530. In use, a voltage polarity provided by the power source 510 can operate the electric motor 502 in a clockwise direction wherein the voltage polarity applied to the electric motor by the battery can be reversed in order to operate the electric motor 502 in a counterclockwise direction. When the electric motor 502 is rotated in one direction, the drive member530 will be axially driven in the distal direction "DD". When the motor 502 is driven in the opposite rotary direction, the drive member 530 will be axially driven in a proximal direction "PD". The handle 100 can include a switch which can be configured to reverse the polarity applied to the electric motor 502 by the power source 510. As with the other forms described herein, the handle 100 can also include a sensor that is configured to detect the position of the drive member 530 and/or the direction in which the drive member 530 is being moved.

Actuation of the motor 502 can be controlled by a firing trigger 540 that is pivotally supported on the handle 100. The firing trigger 540 may be pivoted between an unactuated position and an actuated position. The firing trigger 540 may be biased into the unactuated position by a spring 542 or other biasing arrangement such that when the clinician releases the firing trigger 540, it may be pivoted or otherwise returned to the unactuated position by the spring or biasing arrangement. In at least one form, the firing trigger 540 can be positioned "outboard" of the closure trigger 332 as was discussed above. In at least one form, a firing trigger safety button 550 may be pivotally mounted to the closure trigger 332. The safety button 550 may be positioned between the firing trigger 540 and the closure trigger 332 and have a pivot arm protruding therefrom. When the closure trigger 332 is in the unactuated position, the safety button 550 is contained in the handle 100 where the clinician cannot readily access it and move it between a safety position preventing actuation of the firing trigger 540 and a firing position wherein the firing trigger 540 may be fired. As the clinician depresses the closure trigger 332, the safety button 550 and the firing trigger 540 pivot down wherein they can then be manipulated by the clinician.

As indicated above, in at least one form, the longitudinally movable drive member 530 has a rack of teeth formed thereon for meshing engagement with a corresponding drive gear of the gear reducer assembly 520. At least one form also includes a manually-actuatable "bailout" assembly 560 that is configured to enable the clinician to manually retract the longitudinally movable drive member 530 should the motor 502 become disabled. See FIG. 7. The bailout assembly 560 may include a lever or bailout handle assembly 562 that is configured to be manually pivoted into ratcheting engagement with teeth 532 also provided in the drive member 530. Thus, the clinician can manually retract the drive member 530 by using the bailout handle assembly 562 to ratchet the drive member 530 in the proximal direction PD. U.S. Patent Application Serial No. 12/249,117, filed October 10, 2008, now U.S. Patent No. 8,608,045, entitled POWERED SURGICAL CUTTING AND STAPLING APPARATUS WITH MANUALLY RETRACTABLE FIRING SYSTEM, discloses bailout arrangements and other components, arrangements and systems that may also be employed with the various instruments disclosed herein.

One method of attaching the shaft assembly 1000 to the handle will now be described with reference to FIGS. 6-9. In the illustrated example, the shaft assembly 1000 includes a nozzle assembly 2200 that includes a proximal nozzle assembly 2210 and a distal nozzle assembly 2250. The proximal nozzle assembly 2210 includes a right proximal nozzle segment 2220 and a left proximal nozzle segment 2230. The proximal nozzle segments 2220, 2230 may be attached together by snap lugs, screws, adhesive, etc. The distal nozzle assembly 2250 includes a right distal nozzle segment 2260 and a left distal nozzle segment 2270. The right distal nozzle segment 2260 and the left distal nozzle segment 2270 are coupled together by snap features, lugs, screws, adhesive, etc. The proximal nozzle assembly 2210 and the distal nozzle assembly 2250 may be attached together by adhesive, friction, etc. The nozzle assembly 2200 is journaled on the housing 100 for selective rotation relative thereto about the shaft axis SA. In the illustrated example, the distal nozzle assembly 2250 is provided with an inwardly extending proximal mounting flange 2252 that interfaces with a frame mounting flange 220 formed on frame portion 210 of the frame assembly 200. The proximal nozzle assembly 2210 is formed with fins 2212 to facilitate rotation of the nozzle assembly 2200 about the shaft axis SA.

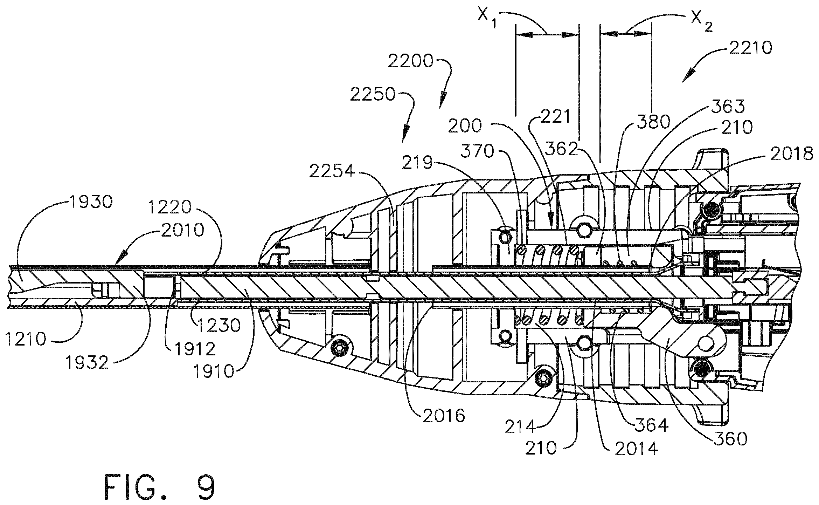

In the illustrated arrangement, the shaft assembly 1000, including the end effector 1500 attached thereto, is rotatable about the shaft axis SA by rotating the nozzle assembly 2200 relative to the handle 100. As can be seen in FIG. 8, for example, the distal nozzle assembly 2250 includes a shaft engagement flange 2254 that extends inwardly through nozzle engagement openings 2016 in a proximal end portion 2014 of the proximal closure tube segment 2010. Such arrangement permits the shaft assembly 1000 to be rotated about the shaft axis SA when the clinician rotates the nozzle assembly 2200. The nozzle engagement openings 2016 are sized to permit axial movement of the proximal closure tube segment 2010 relative to the shaft engagement flange 2254. Still referring to FIG. 8, the distal nozzle assembly 2250 may further include a support sleeve portion 2256 configured to movably support the proximal closure tube segment 2010 therein. In addition, as can be seen in FIG. 9, a proximal end 1226 of the upper spine stay 1220 and a proximal end 1236 of the lower spine stay 1230 are each supported in a spine bearing 1260 that is rotatably supported in the frame assembly 200.

Referring now to FIGS. 6, 8 and 9, the proximal end portion 2014 of the proximal closure tube segment 2010 is movably supported in a closure shuttle 360 that is linked to the second closure link 344 of the closure linkage assembly 340 (FIG. 6). The closure shuttle 360 is slidably supported in the shuttle cavity 214 in the fame assembly 200. The proximal end portion 2014 of the proximal closure tube segment 2010 extends through a U-shaped cradle opening 362 in the closure shuttle 360 to be rotatably supported therein. Such arrangement permits the proximal end portion 2014 of the proximal closure tube segment 2010 to rotate relative to the closure shuttle 360 when the shaft assembly 1000 is rotated relative to the handle 100. In addition, when the clinician depresses the closure trigger 332, the closure shuttle is moved in the distal direction DD within the shuttle cavity 214 and also causes the closure member assembly 2000 in the distal direction to apply closure motions to the end effector 1500.

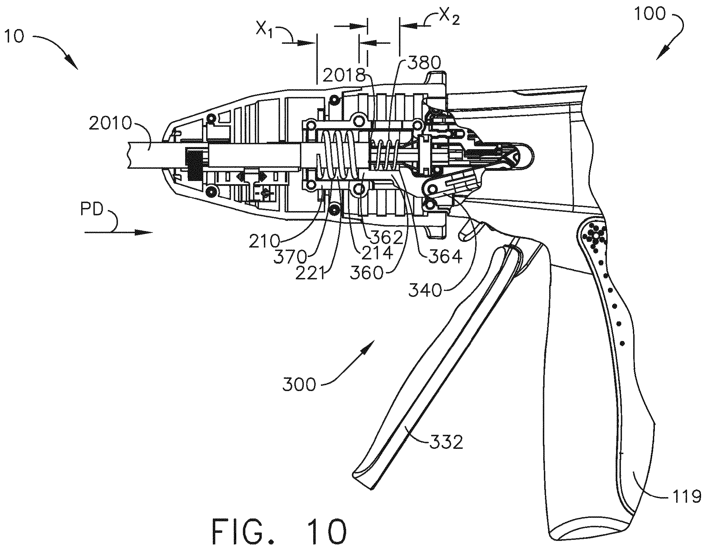

As can be seen in FIGS. 8 and 9, a first spring 370 is journaled on the proximal portion 2014 of the proximal closure tube segment 2010 between the cradle wall 362 of the closure shuttle 360 and a distal end wall 219 formed on the right frame portion 210 (first spring space 221). The axial length of the first spring space is designated X₁. The first spring 370 biases the closure shuttle 360 proximally (arrow PD) into the starting position that corresponds to a fully open position of the anvil. See FIG. 10. In the illustrated example, a proximal flange 2018 is formed on the proximal end portion 2014 of the proximal closure tube segment 2010. The proximal flange 2018 is configured to slidably travel within a closure or second cavity 364 that is formed in the closure shuttle 360 as shown. The axial length of the second cavity or second spring space 363 is designated as X₂. In at least one arrangement, for example, X₁ > X₂. A second closure spring or biasing member 380 is located within the second spring space 363 to bias the proximal flange 2018 on the proximal closure tube segment 2010 in the distal direction DD against the cradle wall 362 of the closure shuttle 360.

During the initial clamping of the target tissue between the anvil 1610 and the surgical staple cartridge 1540, the closure drive system 300 must apply a sufficient amount of axial closure force to the anvil 1610 to pivot the anvil 1610 to a closed position and retain it in that position throughout the staple forming process. The term "closure procedure" in at least one application refers to the process of moving the anvil from a fully opened position to a closed position on the target tissue and retaining the anvil in the closed position until the staple forming process has been fully completed and the anvil is ready to be reopened to release the target tissue from the end effector. The amount of closure force required to close the anvil and retain it in a closed position can vary during the stapling process due to "tissue creep". For example, as the anvil compresses the target tissue, fluid within the clamped target tissue can "creep" or migrate within the tissue and even flow to adjacent unclamped tissue. Further tissue creep may be experienced as the knife portion 1950 is driven through the clamped target tissue. Thus, tissue creep can also affect the amount of firing force required to cut the target tissue and fire the staples within the staple cartridge. As the knife portion 1950 nears the end of its stroke, the amount of required firing force may be reduced because the creeping fluid has completed the migration into the adjacent unclamped tissue.

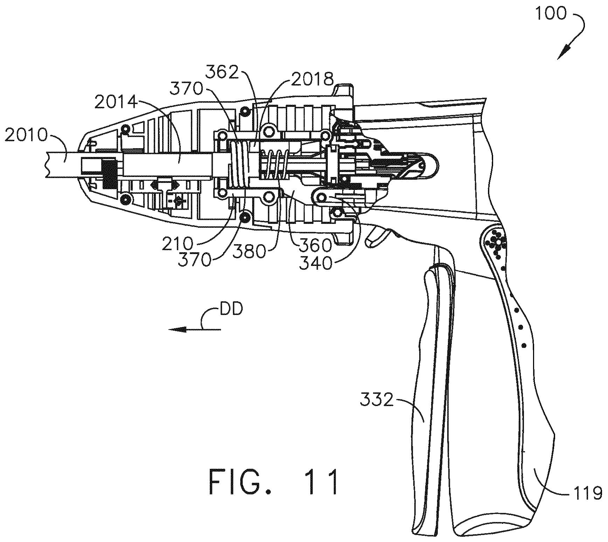

FIG. 10 illustrates the closure drive system 300 in an unactuated orientation. As can be seen in FIG. 10, the first spring 370 has biased the closure shuttle 360 proximally in the PD direction within the shuttle cavity 214 in the frame assembly 200 to its proximal-most or starting position. As a result of the closure shuttle 360 being attached to the closure trigger 332 by the closure linkage assembly 340, the closure trigger 332 is pivoted into the unactuated starting position. When the closure drive system 300 is in that unactuated position, the anvil 1610 is in a fully open position. In at least one example, the first spring 370 is "weaker" than the second spring 380. Stated another way, the second spring 380 is stiffer than the first spring 370. That is the spring constant K₁ of the first spring 370 is less than the spring constant K₂ of the second spring 380. Thus, K₂ > K₁. In alternative arrangements, K₂ < K₁. In some arrangements , for example, K₂ may be up to ten times greater than K₁. In other arrangements K₂/K₁ > 1. The clinician initiates the closure process by depressing the closure trigger 332 towards the pistol grip 119 of the housing 100. This action starts to move the closure shuttle 360 in the distal direction DD which starts to compress the first spring 370. As the closure shuttle 360 starts moving distally, the closure shuttle 360 also starts moving the proximal closure tube segment 2010 distally. As the proximal closure tube segment 2010 moves distally, the entire closure member assembly 2000 (of which the proximal closure tube segment 2010 is a part) moves distally to apply closure motions to the anvil 1610. As the clinician continues to pivot the closure trigger 332 toward the pistol grip 119, the closure shuttle 360 continues to move distally and compresses the first spring 370. As the anvil 1610 starts to close, the amount of closure forces required may begin to increase as the anvil 1610 begins to compress the target tissue. As the target tissue begins to compress, the fluids contained therein may begin to creep within the target tissue which can directly affect the amount of closure forces required to completely close the anvil. As the resistance to closure increases, the closure member assembly 2000 can move in the proximal direction PD against the closure force applied by the second spring 380 to the proximal closure tube segment 2010. FIG. 11 depicts the closure drive system 300 in a fully closed position that corresponds to a fully closed position of the anvil 1610. Thus, the ultimate amount of closure force applied to the anvil 1610 through the closure member assembly 2000 can vary due to the ability of the second spring 380 to compress in response to the closure resistance experienced by the anvil during the closure procedure. Thus, the anvil can "progressively close" as it experiences changes in the amount of resistance created by the target tissue. This may also be referred to herein as generating a "progressive closure force".

Once the jaws 1510, 1600 are closed onto the target tissue and locked in that position, the clinician may then institute the firing process by depressing the firing trigger 540 which causes the knife bar 1910 to drive the knife portion 1950 through the clamped target tissue. As the knife portion 1950 is driven distally through the end effector 1500, the knife blade 152 cuts through the target tissue. In addition, in at least one arrangement, the knife portion 1950 engages and distally drives a camming assembly, sometimes referred to as a wedge sled 1970 that is slidably supported in the surgical staple cartridge 1540. As the wedge sled 1970 is driven distally through the staple cartridge 1540, the cams formed on the wedge sled 1970 camming engage staple drivers (not shown) that are movably supported within the staple cartridge 1540. Each staple driver may support one or more surgical staples thereon. The staple drivers are commonly supported in axially lines located on each side of an elongated slot that is formed in the staple cartridge. As the wedge sled 1570 contacts the staple drivers, they are driven upward (toward the closed anvil) 1610 thereby driving the staple(s) supported thereon through the target tissue and into forming contact with the staple forming undersurface 1614 of the anvil body 1612. The wedge sled 1570 is position distal to the tissue cutting knife blade, so the staples are deployed through the target tissue before the target tissue is cut.

Further, as the knife portion 1950 is driven through the target tissue, tabs or flanges formed in the knife portion engage the anvil 1610 as well as the elongated channel 1510 and retain the anvil 1610 and channel 1510 closed and spaced at a desired spacing arrangement during the stapling process. While such distal advancement of the knife portion may reduce the amount of closure force required from the closure system, a significant amount of "firing forces" must be generated by the firing system to push the knife portion 1950 through the target tissue and to overcome the resistive forces and friction of the system as the wedge sled actuates the staple drivers. Thus, the firing system must be able to generate sufficient firing forces and the firing system components must be sufficiently robust to effectively accommodate such forces while also being flexible enough to accommodate articulation of the end effector. These design requirements of the closure and firing systems may also be exacerbated by the type and composition of the target tissue. Further, the components of these systems must be sufficiently small enough to be inserted through the small cannulas of trocars.

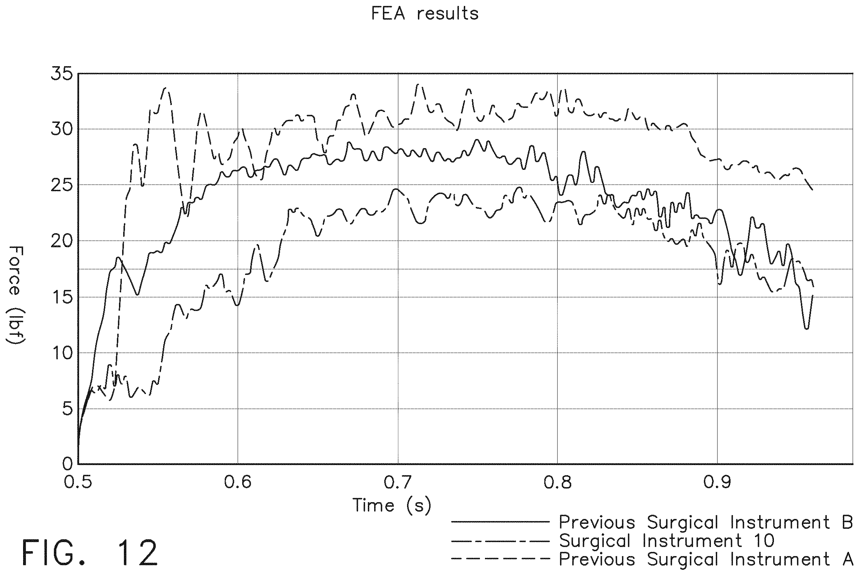

FIG. 12 is a graphical comparison between a the surgical instrument 10 that employs the progressive closure drive system 300 described above and two previous surgical instruments A and B that employ different closure drive arrangements. Previous surgical instrument A employs a closure drive system that is directly linked to the closure actuator or closure trigger. While previous surgical instrument A does employ a spring for biasing the closure system to an unactuated position, previous surgical instrument A does not employ a second biasing member like surgical instrument 10 described above. In addition, the anvil of surgical instrument A lacks a camming surface or surfaces like anvil 1610 described above. Instead, the anvil of surgical instrument A has a hard edge that is arranged for contact by the closure member or tube. As the closure member contacts that hard edge, the anvil is pivoted closed.

Still referring to FIG. 12, previous surgical instrument B is similar in some aspects to previous surgical instrument A in that surgical instrument B has a first biasing member for biasing the closure system to an unactuated position. However, surgical instrument B does not employ a second biasing member like instrument 10 described above. The anvil of surgical instrument B, however, does employ camming surfaces that are configured to be contacted by the closure member to pivot the anvil to a closed position. FIG. 12 is a plot showing the amount of time to complete the closure procedure vs the amount of closure forces required during the procedure for each of the three surgical instruments: surgical instrument 10, surgical instrument A and surgical instrument B. As can be seen in FIG. 12, the amount of closure forces required throughout the closure procedure for surgical instrument 10 is less than the closure forces required by surgical instruments A and B. While the closure forces needed by surgical instrument B are less than the closure forces required by surgical instrument A, the closure forces required by surgical instrument 10 are considerably less than the closure forces required by both A and B throughout the closure process.

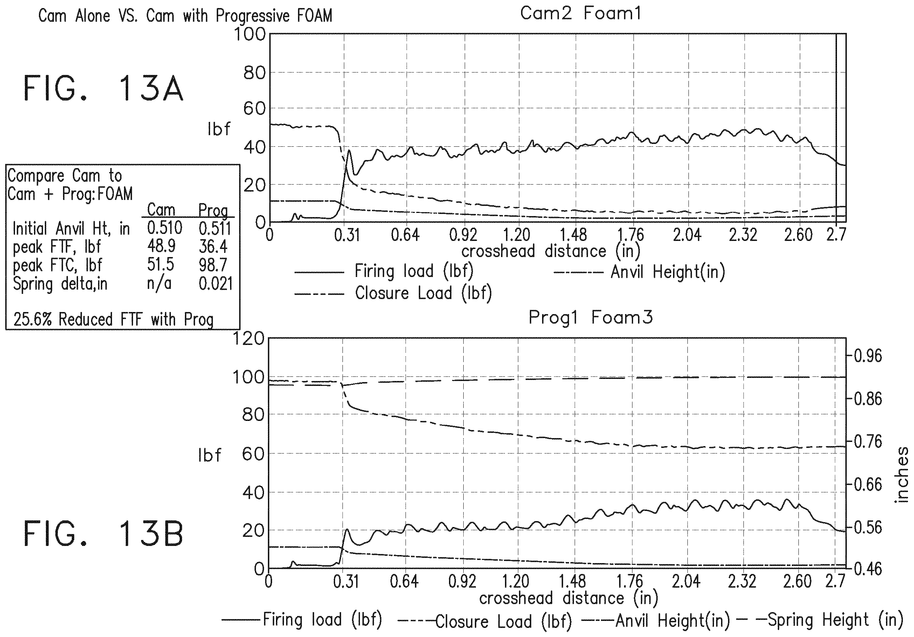

FIGS. 13A and 13B compare the force to fire (FTF), the force to close (FTC) experienced by surgical instrument 10 and surgical instrument B (with the camming surfaces on the anvil) as the firing member or knife travels through the anvil from a proximal-most starting position to a distal-most ending position in the anvil (crosshead distance). FIG. 13A illustrates the FTF, FTC and the anvil height during the firing procedure for the surgical instrument B. FIG. 13B illustrates the FTF, FTC, anvil height and spring height of surgical instrument 10. As can be seen from reference to FIGS. 13A and 13B, the initial anvil height for surgical instrument B was 0.0510 inches (1,2954 mm) and the initial anvil height for surgical instrument 10 was 0.511 inches (12,9794 mm). The peak closure force FTC in pounds for surgical instrument B was 51.5 pounds (23,4 kg) and for surgical instrument 10 was 98.7 pounds (44,8 kg). The peak FTF in pounds for surgical instrument B was 48.9 pounds (22,2 kg) and for surgical instrument 10 was 36.4 pounds (16,5 kg). Thus, surgical instrument 10 experienced a 25.6 % reduction in the amount of closure forces required by surgical instrument 10. This reduction in the amount of firing force required may allow the firing system components to be fabricated from lighter and or smaller component arrangements.

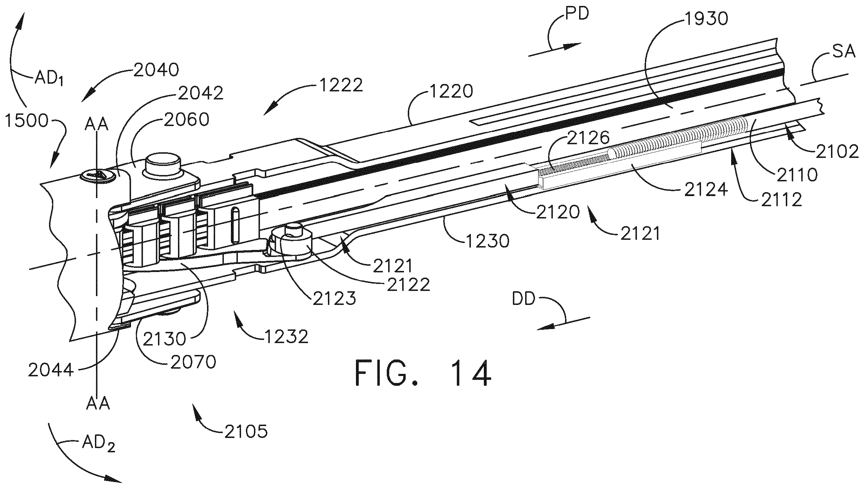

As indicated above, the surgical instrument 10 includes an articulation drive system 2100 that is configured to selectively articulate the surgical end effector 1500 relative to the shaft assembly 1000 about an articulation axis AA that is transverse to the shaft axis SA. See FIG. 14. The articulation drive system 2100 includes an articulation drive assembly 2102 that comprises a proximal articulation driver 2110 that is coupled to an intermediate articulation driver 2120 that is pivotally coupled to a distal articulation link 2130 that is attached to the proximal end of the elongated channel 1520. See FIG. 2. In one example, the distal articulation link 2130 is attached to the intermediate articulation driver 2120 on one side of the shaft axis SA. The distal articulation link 2130 is attached to the elongated channel 1520 on the opposite side of the shaft axis SA so that the distal articulation link 2130 extends transversely across the shaft axis SA. In the illustrated example, the joint between the proximal articulation driver 2110 and the intermediate articulation driver 2120 may also function as an articulation lock assembly 2121 that serves to retain the surgical end effector 1500 in an articulated position after the articulation motion applied to the proximal articulation driver 2110 is discontinued. In the illustrated example, a distal end 2112 of the proximal articulation driver 2110 is threaded. The threaded distal end 2112 of the proximal articulation driver 2110 is in threaded engagement with a threaded socket 2126 in a proximal end 2124 of the intermediate articulation driver 2120. Rotation of the proximal articulation driver 2110 in a first rotary direction will cause the intermediate articulation driver 2120 to axially move in a first or distal direction DD. Movement of the intermediate articulation driver 2120 in the distal direction DD will cause the surgical end effector 1500 to pivot about the articulation axis AA in a first articulation direction AD₁. Rotation of the proximal articulation driver in a second rotary direction will cause the intermediate articulation driver 2120 to move in a second or proximal direction PD. Axial movement of the intermediate articulation driver 2120 in the proximal direction PD will cause the surgical end effector 1500 to pivot about the articulation axis AA in a second articulation direction AD₂.

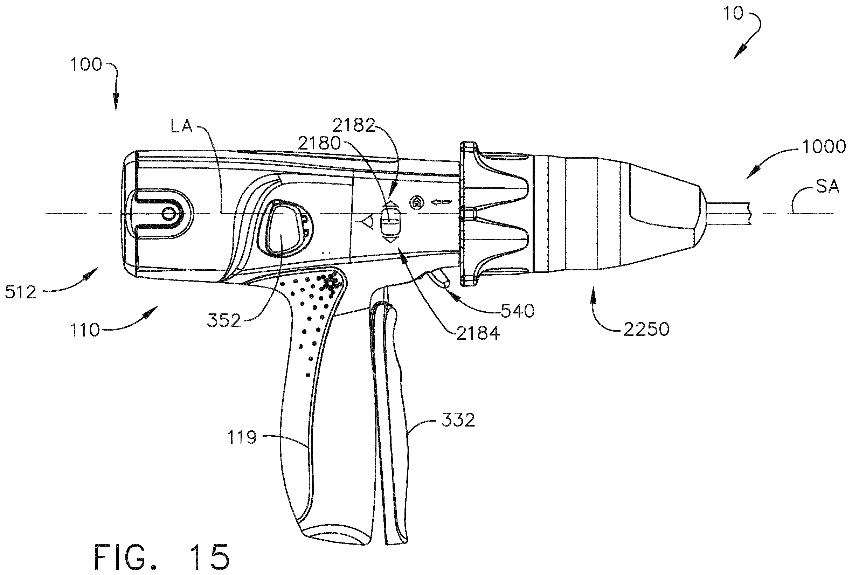

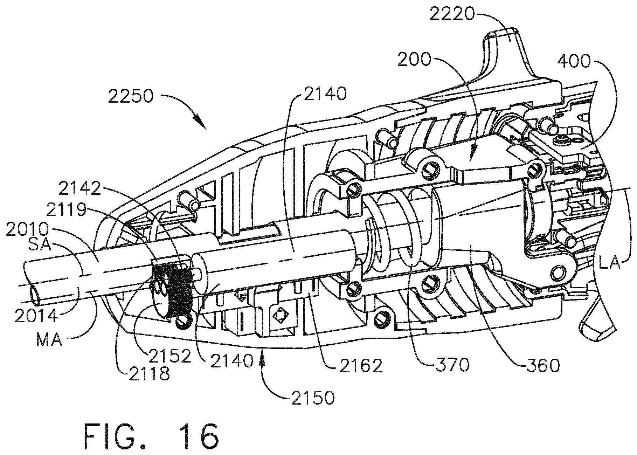

In the illustrated example, housing 100 or handle 110 defines a longitudinal axis LA. See FIGS. 15 and 16. As indicated above, the shaft assembly 1000 also defines a shaft axis SA. The shaft axis SA and the longitudinal axis LA may be coaxial. The longitudinal the articulation drive system 2100 includes an articulation motor 2140 that is mounted within a distal nozzle assembly 2250 for rotational travel therewith in an orbit about the longitudinal axis LA when the user rotates the nozzle assembly 2250 relative to the housing 100. The distal nozzle assembly 2250 may also be referred to herein as a "shaft rotator assembly" that is configured to rotatably couple the shaft assembly 1000 to the housing 100. In one arrangement, the articulation motor 2140 includes a gear configuration that includes a motor output gear 2142. The motor output gear 2142 rotates about motor axis MA that is parallel to and offset from the longitudinal axis LA. See FIG. 16. In the illustrated example, the motor output gear 2142 is in meshing engagement with a control switch gear 2152 of a motor switch system 2150. The control switch gear 2152 is in meshing engagement with a proximal articulation drive gear 2118 that is formed on a proximal end 2116 of the proximal articulation driver 2110. Thus, rotation of the motor output gear 2142 in one direction will result in rotation of the proximal articulation driver 2110 in the first direction which will cause the intermediate articulation driver 2120 in the distal direction and cause the surgical end effector 1500 to articulate in the first articulation direction AD₁. Likewise rotation of the articulation motor in an opposite rotary direction will cause the proximal articulation driver 2110 to rotate in a second rotary direction and thereby cause the intermediate articulation driver 2120 to move in the proximal direction PD. Movement of the intermediate articulation driver 2120 in the proximal direction PD will cause the surgical end effector 1500 to articulate in the second articulation direction AD₂. In various embodiments, the articulation motor 2140 and gear configuration may, for example, be less than 12mm in diameter and less than 1.5 inches (38,1 mm) long. The articulation motor 2140 may be of a brushed design with less than 1.5 watt power output. In at least some embodiments, the power output of the motor 2140 is approximately .75-1.0 watts. The gear configuration may be supported by the motor housing or it may be separate from the motor housing. In at least one example, the gear configuration has a 100:1 reduction although gear configurations with higher reduction ratios may be employed. In alternative arrangements, other motor configurations may be employed.

With respect to the articulation lock 2121, the threads on the distal end 2112 of the proximal articulation driver 2110 may, for example, comprise a # 2 screw thread with either a fine (e.g., 64 threads/inch) or a coarse (less than 64 threads/inch) (1 inch = 25,4 mm) provides sufficient mechanical advantage for the articulation motor 2140 to cause articulation of the surgical end effector 1500 while also functioning as a lock to prevent movement of the surgical end effector 1500 after the articulation motor 2140 has been de-energized. As can also be seen in FIG. 2, the proximal articulation driver 2110 may be provided with a support shoulder portion 2119 that has a larger diameter than the adjacent portions of the of the proximal articulation driver 2110. The larger shoulder 2119 slidably interfaces with the spine shaft 1210 for additional support when in the locked position.

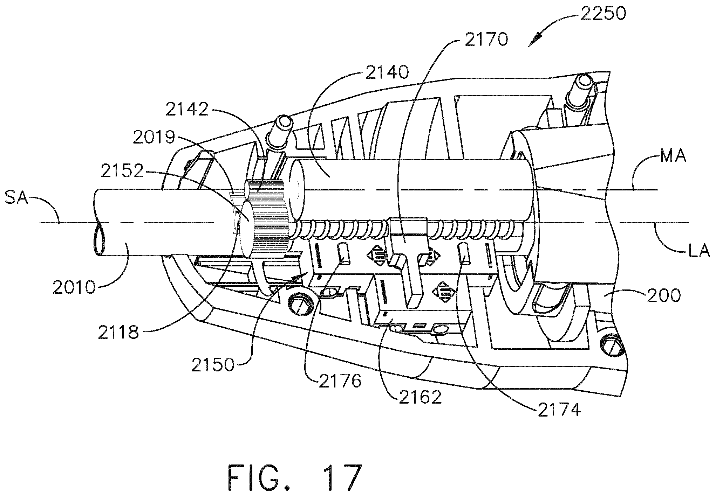

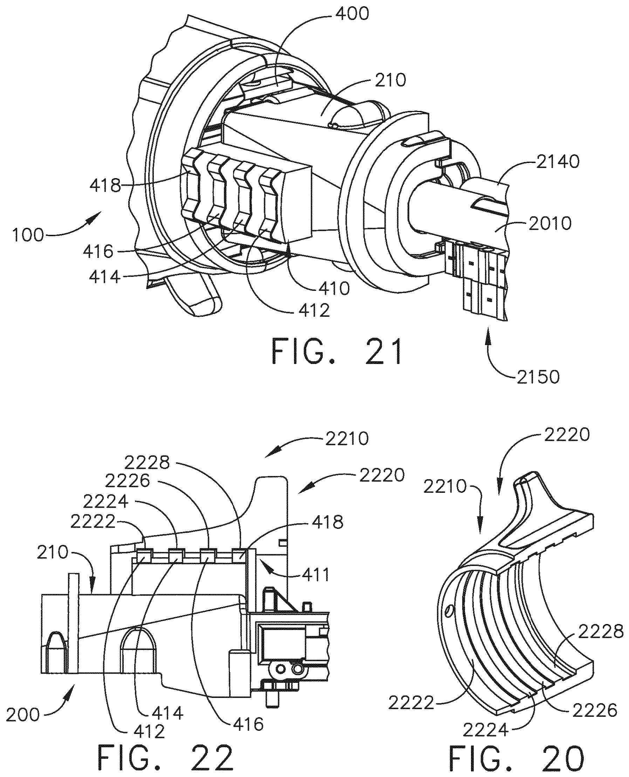

Still referring to FIGS. 16-19, the motor switch system 2150 includes a switch traveler 2170 that is threaded onto a switch drive screw 2160 that is attached to the control switch gear 2152. Rotation of the motor output gear 2142 will cause the control switch gear 2152 to rotate which ultimately causes the switch drive screw 2160 to rotate. Rotation of the switch driver screw 2160 results in the axial movement of the switch traveler 2170 relative to a switch housing 2162 mounted in the distal nozzle assembly 2250. The switch housing 2162 operably supports a plurality of limit switches that are in communication with the control circuit board 400 that supported in or otherwise associated with the housing 100 as will be further discussed below. See FIG. 16. In the illustrated example, three limit switches are employed: a central limit switch 2172, a proximal limit switch 2174 and a distal limit switch 2176. The switches 2172, 2174, 2176 are wired to a series of circuit traces or conductors 2222, 2224, 2226, 2228 that are mounted within the proximal nozzle assembly 2210. See FIG. 20. Circuit traces 2222, 2224, 2226 and 2228 are wired or electrically coupled to the articulation motor 2140 and switches 2172, 2174, 2176 by wires or flexible circuit conductors (not shown). Turning to FIGS. 21 and 22, a contact block 410 is fixedly mounted to the frame assembly 200 and includes contacts 412, 424, 416 and 418 that correspond respectively to circuit traces 2222, 2224, 2226 and 2228. Contacts 412, 414, 416, 418 are wired to the control circuit board 400. As the proximal nozzle assembly 2210 is rotated relative to the housing 100 about the shaft axis SA, power/control signals may be provided between the control circuit board 400 and the articulation motor 2140 and switches 2172, 2174, 2176 through a slip joint assembly 411 which comprises the circuit traces 2222, 2224, 2226, 2228 and contacts 412, 414, 416, 418 to facilitate rotation of the articulation motor 2140 and the control switch assembly relative to the housing 100 about the shaft axis SA. Articulation control switches 2180 are mounted on each side of housing 100 and are used to control the rotation of the articulation motor 2140. See FIG. 15. Switches 2180 may comprise "rocker-type" switches that, when depressed in one direction (arrow 2182), the articulation motor 2140 rotates the motor output gear 2142 in one rotary direction and when a switch 2180 is depressed in an opposite direction (arrow 2184) the articulation motor rotates the motor output gear 2142 in an opposite rotary direction.

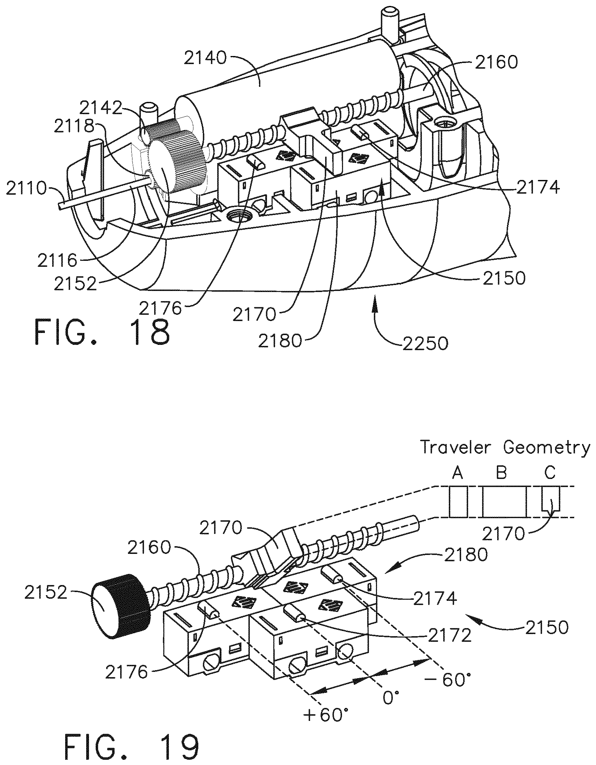

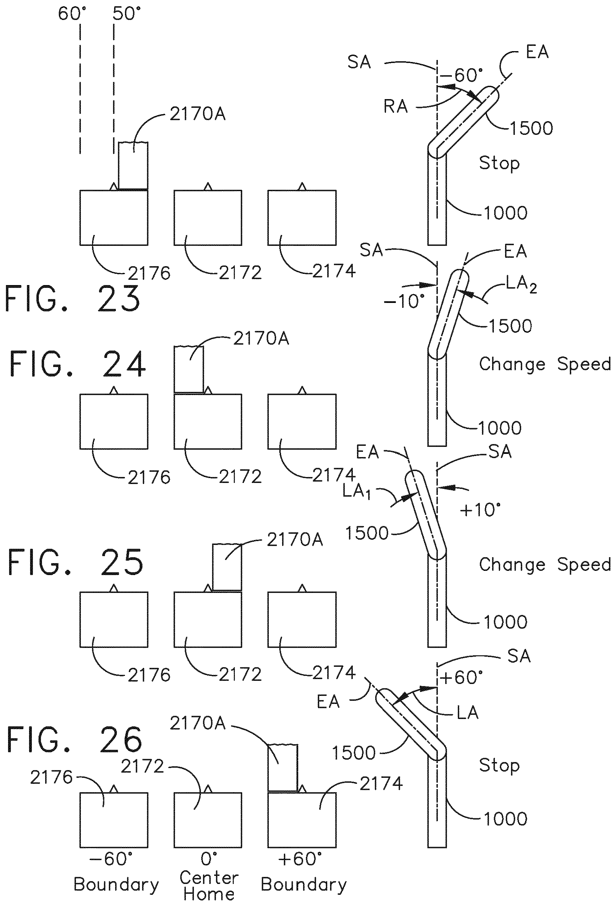

Turning now to FIGS. 19 and 23-26, the positions of switches 2172, 2174, 2176 relative to the path of the switch traveler 2170 serve to define the range of articulation of the surgical end effector 1500. Switch 2172 comprises the central or home switch that corresponds to the unarticulated position of the surgical end effector 1500. When in that position, a central end effector axis EA is generally aligned with the shaft axis SA. When in this position, for example, the end effector 1500 may be inserted through or removed from a trocar cannula. Switch 2174 corresponds to a -60° left articulation boundary and switch 2176 corresponds to a +60° right articulation boundary. The -60 ° left articulation boundary may also be referred to herein as a "first maximum articulated position" of the surgical end effector 1500 located on a "first side" or left side of the shaft axis SA. The left -60° angle (LA in FIG. 26) may also be referred to as a "first maximum articulation angle" and comprises the angle between the end effector axis EA and the shaft axis SA when the surgical end effector 1500 is in the first maximum articulated position. Similarly, the +60° right articulation boundary may also be referred to herein as a "second maximum articulated position" of the surgical end effector 1500 located on a "second side" or right side of the shaft axis SA. The right +60° angle (RA in FIG. 23) may also be referred to as a "second maximum articulation angle" and comprises the angle between the end effector axis EA and the shaft axis SA when the surgical end effector 1500 is in the second maximum articulated position. Thus, in the illustrated example, the positions of switches 2174 and 2176 establish the maximum articulation positions for each articulation direction (left and right). In alternative arrangements, only two switches (2174, 2176) may be employed. The switches 2172, 2174, 2176 may comprise mechanical switches, Hall Effect switches, etc.

In at least one example, the central home switch 2172 may also be used to slow the articulation motor 2140 down prior to crossing the home or unarticulated position to allow the user to more easily determine when the end effector 1500 is aligned with the shaft assembly 1000 which would facilitate removable through a trocar, for example.

In on example, the geometric shape of the switch traveler in the region that is configured to engage the switches 2172, 2174, 2176 is selected to have a width such that it engages the switches at "X" degrees from home and is able to remain in contact with the switch from =X degrees to -X degrees. In at least one example, X = approximately 10 degrees, but X could be other values as well. FIGS. 23-26 illustrate the relationship between the switch traveler 2170 and switches 2172, 2174, 2176 and the articulated position of the surgical end effector 1500. For example, in FIG. 25, when the switch traveler 2170 is in that position, the surgical end effector 1500 may be in a first articulated position on the first side of the shaft axis SA, wherein the surgical end effector axis EA is positioned at a first articulation angle LA₁ relative to the shaft axis SA. Likewise, when the switch traveler 2170 is in the position illustrated in FIG. 24, for example, the surgical end effector 1500 may be in a second articulated position located on a second side of the shaft axis SA, wherein the surgical end effector axis EA is positioned at a second articulation angle LA₂ relative to the shaft axis SA. In at least one arrangement, LA₁ = LA₂ = approximately 10°, for example.

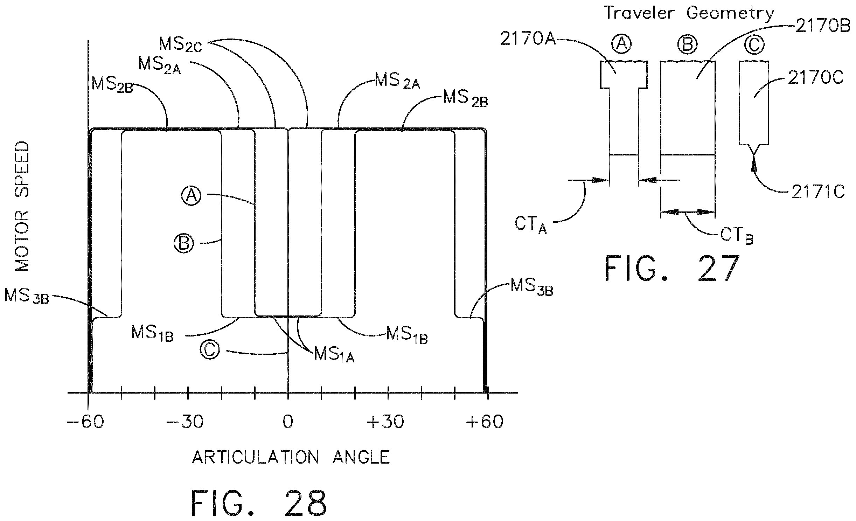

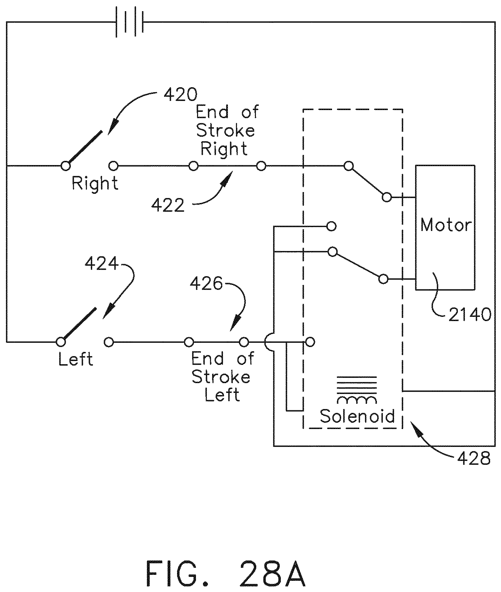

FIG. 27 provides alternative geometrical shapes of the switch traveler 2170 when viewed from the free end of the switch traveler 2170 that is configured to interact with switches 2172, 2174, 2176. FIG. 28 is a graphical comparison of motor speeds (for each geometrical shape 2170A, 2170B, 2170C) to articulation angle. Switch traveler 2170A is also depicted in FIGS. 23-26. 2170C has an actuator point 21701C formed thereon for more precise actuation of the switches 2172, 2174, 2176. As can be seen in FIG. 28, the motor speed MS_{2C} only drops off when the actuator point 2171C is in actuation contact with one of the switches 2172, 2174, 2176 which correspond to articulation angles of -60°, 0° , +60°. 2170A has a cross-sectional thickness CT_{A} which results in a first motor speed MS_{1A} for an articulation angle range between -10° to +10°. For articulation angles between -10° to -60° and +10° to +60°, the articulation motor 2140 operates at a second motor speed MS_{2A} that is greater than MS_{1A}. 2170B has a cross-sectional thickness CT_{B} which is greater than the cross-sectional thickness CT_{A} and results in a first motor speed MS_{1B} for an articulation angle range between -20° to +20°. For articulation angles between -20° to -50° and +20° to +50°, the articulation motor 2140 may operate at a second motor speed MS_{2B} that is greater than MS_{1B}. For articulation angles between -50 to -60 and +50 to +60 the articulation motor 2140 may operate at a third motor speed MS_{3B}. In the illustrated example, MS_{3B} = MS_{1B}.

In various arrangements, the control circuit board 400 may include switches 420, 422, 424, 426 that define the articulation limits as well as a latchable or relay switch 428 that controls the center or home position of the end effector. See FIG. 28A. In such arrangement, as the articulation motor 2140 drives the end effector across the straight or home position, the latchable or relay switch 428 could be tripped which would deactivate the articulation motor 2140. Releasing the articulation control switch 2180 could activate a bypass relay which could deactivate the center or home switch 2172 and pressing the control switch 2180 again could allow the articulation to continue through the home position. See FIG. 28A.

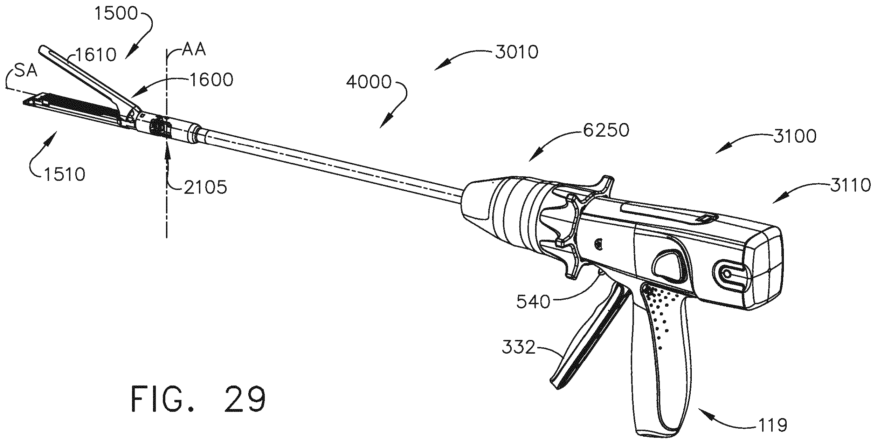

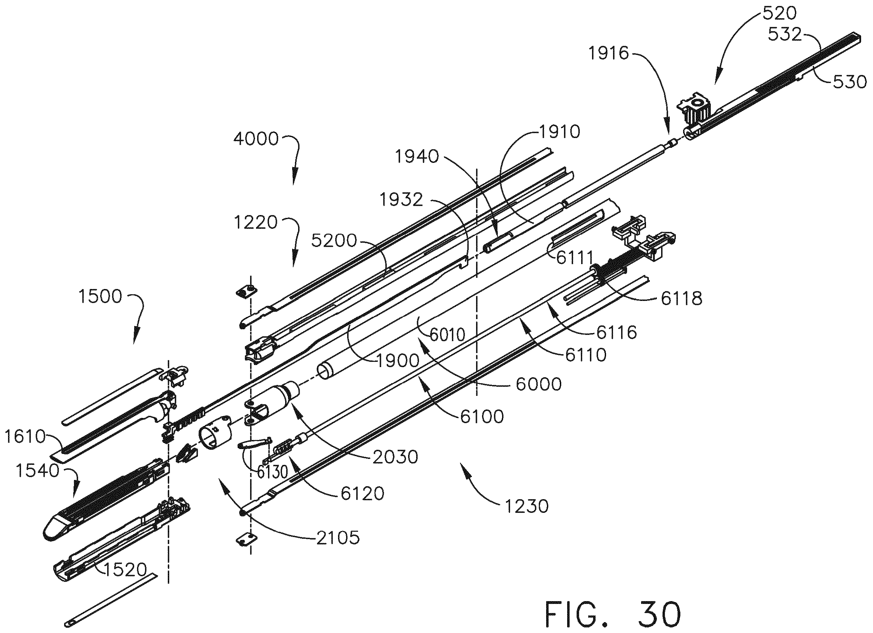

FIGS. 29-33 illustrate a surgical instrument 3010 that is similar to surgical instrument 10 except for the differences discussed below. Surgical instrument 3010 comprises a shaft assembly 4000 that is similar to shaft assembly 1000. The elongated shaft assembly 4000 is operably coupled to a housing 3100 that comprises a handle 3110. Housing 3100 may be similar to housing 100 described above, except for the differences discussed below. Portions of an example of elongated shaft assembly 4000 are depicted in FIG. 30. Those elements that are the same or identical to the elements of shaft assembly 1000have been identified with like element numbers. The surgical instrument 3010 comprises an end effector 1500 (as described above) that is movably coupled to a spine assembly 5200. The spine assembly 5200 is configured to, one, slidably support a firing member assembly 1900 therein and, two, slidably support a closure member assembly 6000 which extends around the spine shaft 5200. The closure member assembly 6000 is similar to closure member assembly 2000 described above, except for the differences discussed below. The closure member assembly 6000 comprises a proximal closure tube or closure member segment 6010 that is similar to proximal closure tube segment 2010. The proximal closure tube segment 6010 interfaces with a closure shuttle that is operably supported in housing 3100 in the manner discussed above or described in various references. The surgical instrument also comprises a firing drive system 500 that is configured to apply firing motions to the firing member assembly 1900 in the shaft assembly 4000. As described above, the firing drive system 500 includes a motor driven longitudinally-movable drive member 530. Actuation of the motor can be controlled by a firing trigger 540 that is pivotally supported on the handle 3110.

As was also discussed above, the shaft assembly 4000 further includes a firing member assembly 1900 that is supported for axial travel within the spine shaft 1210. The firing member assembly 1900 includes an intermediate firing shaft portion 1910 that is configured for attachment to a distal cutting portion or knife bar 1930. The intermediate firing shaft portion 1910 may include a longitudinal slot 1912 in the distal end thereof which can be configured to receive a tab 1932 on the proximal end of the distal knife bar 1930. The longitudinal slot 1932 and the proximal end tab 1932 can be sized and configured to permit relative movement therebetween and can comprise a slip joint 1940. The slip joint1940 can permit the knife bar 1930 to move axially relative to the intermediate firing shaft portion 1910 to accommodate articulation of the end effector 1500, for example. The knife bar 1910 includes a knife portion 1950.

In the illustrated example, the surgical end effector 1500 is selectively articulatable about a articulation axis AA by an articulation drive system 6100. In at least one example, articulation drive system 6100 is configured to converts a linear stroke of the intermediate firing shaft portion 1910 to a rotary articulation motion that can be employed to articulate the surgical end effector 1500 relative to the elongated shaft assembly 4000. In one form, the articulation drive system 6100 includes a proximal articulation driver 6110 that is operably coupled to an intermediate articulation driver 6120 that is pivotally coupled to a distal articulation link 6130 as will be discussed in further detail below.

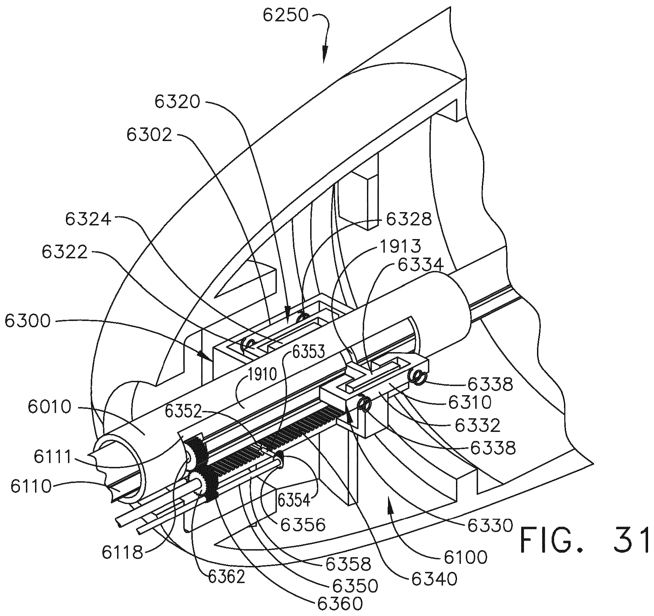

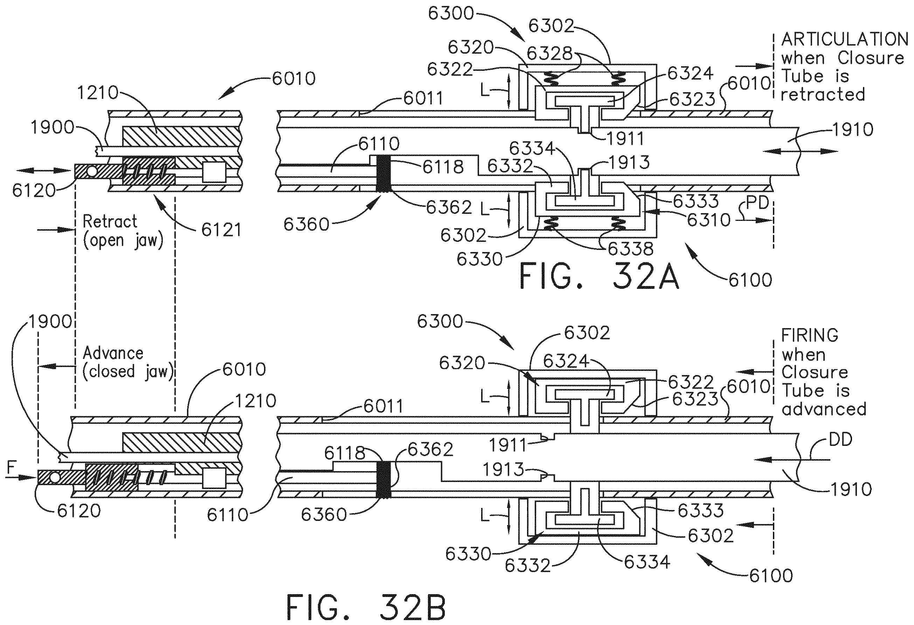

Turning now to FIGS. 31, 32A and 32B, in at least one form, the articulation drive system comprises an articulation clutch assembly 6300 that is operably supported in the nozzle assembly 6250 that is rotatably coupled to the housing 3100 as was described above. In the illustrated example, the articulation clutch assembly 6300 comprises a clutch mounting member 6302 that is fixedly attached to the nozzle assembly 6250. Movably supported within the clutch mounting member 6302 is a clutch shifter assembly 6310. In one form, the clutch shifter assembly 6310 includes first and second laterally opposed lock assemblies 6320, 6330 that are movable in directions that are transverse to the shaft axis SA. This transverse locking movement is represented by arrows L in FIGS. 32A and 32B. In the illustrated example, the first lock assembly 6320 includes a first movable lock support 6322 that supports a first clutch lock 6324. The first clutch lock 6324 is configured to lockingly engage a first lock groove 1911 in the intermediate firing shaft portion 1910. First biasing members or springs 6328 serve to bias the first lock assembly 6320 towards the intermediate firing shaft portion 1910 to cause the first clutch lock 6324 to lockingly engage the first lock groove 1911 in the intermediate firing shaft portion 1910 as shown in FIG. 32A.

Similarly, the second lock assembly 6330 includes a second movable lock support 6332 that supported a second clutch lock 6334. The second clutch lock 6334 is configured to lockingly engage a second lock groove 1913 in the intermediate firing shaft portion 1910. Second biasing members or spring 6338 serve to bias the second lock assembly 6330 towards the intermediate firing shaft portion 1910 to cause the second clutch lock 6334 to lockingly engage the second lock groove 1913 in the intermediate firing shaft portion 1910 as shown in FIG. 32A.

In the illustrated example, the articulation clutch assembly 6300 further comprises a clutch driver rack 6340 that is attached thereto so as to axially move with the first and second clutch locks 6324, 6334, while permitting the first and second clutch locks to move transversely relative to the shaft axis between "locked" or "engaged" positions and "unlocked" or "disengaged" positions. See FIG. 31. The clutch driver rack 6340 is in meshing engagement with a clutch gear assembly 6350 that includes a rack gear 6352 that is in meshing engagement with the clutch driver rack 6340 and is attached by a first transfer shaft 6353 to first and second meshing clutch bevel gears 6354, 6356. Still referring to FIG. 31, the second clutch bevel gear 6356 is attached to a second transfer shaft 6358 that has a transfer drive gear 6360 attached thereto. The transfer drive gear 6360 is in meshing engagement with an idler gear 6362 that is in meshing engagement with a proximal articulation drive gear 6118 formed on a proximal end of the proximal articulation driver 6110. Thus, axial movement of the clutch driver rack 6340 will ultimately result in the rotation of the proximal articulation driver 6110 through the gear arrangements described above. Such rotation of the proximal articulation driver 6110 in the first direction will cause the intermediate articulation driver 2120 to move in the proximal direction and cause the surgical end effector 1500 to articulate in a first articulation direction. Likewise, axial movement of the clutch driver rack 6340 in proximal direction PD will cause the proximal articulation driver 6110 to rotate in a second rotary direction and thereby cause the intermediate articulation driver to move in the distal direction DD. Movement of the intermediate articulation driver 2120 in the distal direction DD causes the surgical end effector 1500 to articulate in a second articulation direction.

FIG. 32A, illustrates the articulation clutch assembly 6300 in an engaged or locked position with the intermediate firing shaft portion 1910 of the firing shaft assembly 1900. When in that position, axial movement of the intermediate firing shaft portion 1910 (caused by actuating the firing trigger 540 as described above), will result in the axial movement of the articulation clutch driver rack 6340 and ultimately result in the articulation of the surgical end effector as described herein. The first biasing members 6328 and the second biasing members 6338 bias the clutch assembly 6300 in this normally engaged position. FIG. 32A illustrates the proximal closure tube segment 6010 in its proximal-most , unactuated position that results in the jaws 1610, 1610 being in the open position. Thus, with the jaws in the open position, actuation of the firing drive system will result in the articulation of the surgical end effector 1500.

After the clinician has articulated the surgical end effector in the desired articulated position, the clinician may then commence the jaw closing process by actuating the closure system by actuating the closure system by depressing the closure trigger 332. As can be seen in FIGS. 32A and 32B, the clutch assembly protrudes through a clutch opening 6011 in the proximal closure tube segment 6010. As was discussed above, depressing the closure trigger 332 causes the proximal closure tube segment 6010 to axially move in the distal direction DD. As the proximal closure tube segment 6010 moves distally, it contacts a first cam surface 6323 on the first movable lock support 6322 and a second cam surface 6333 on the second movable lock support 6332 to move the first and second lock supports 6322, 6332 transversely in opposite directions away from the shaft axis. Such movement of the first and second movable lock supports 6322, 6332 causes the first and second clutch locks 6324, 6334 to disengage the first lock groove 1911 and the second lock groove 1913 in the intermediate firing shaft portion 1910 as shown in FIG. 32B. Thus, the articulation clutch assembly 6300 has disengaged the articulation system from the firing drive system and the clinician can now actuate the firing drive system while the surgical end effector remains articulated.

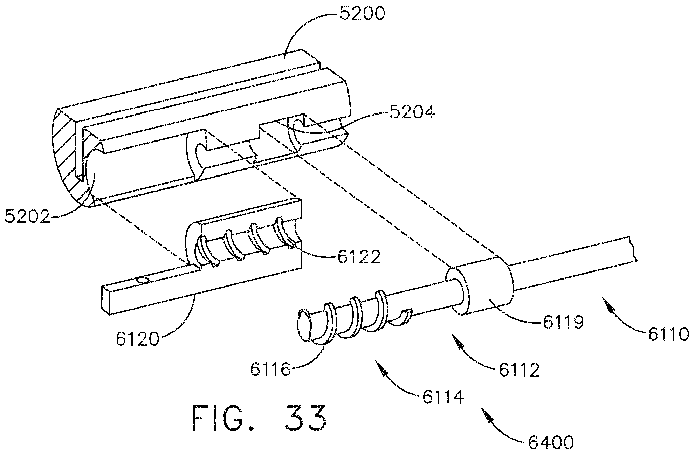

In the illustrated example, the articulation drive system also include an articulation lock arrangement 6400 for retaining the surgical end effector in an articulated position after the articulation drive system has been deactivated. More particularly and with reference to FIG. 33, the distal end portion 6112 of the proximal articulation driver 6110 includes a threaded portion 6114 formed thereon. In one arrangement the threaded portion 6114 comprises an ACME thread that can be dropped into a threaded passage segment 6122 in intermediate articulation driver 6120. The intermediate articulation driver 6120 is slidably supported in a distal driver cavity 5202 in the spine shaft 5200. As indicated above, the intermediate articulation driver 6120 is pivotally pinned to the distal articulation link 6130. The distal articulation link 6130 extends transversely across the shaft axis SA and is pivotally pinned to a proximal end of an elongated channel 1520. As can be seen in FIG. 33, a shoulder 6119 is formed on the proximal articulation driver 6110 and is rotatably received within a shoulder cavity 5204 in the spine shaft 5200. Rotation of the proximal articulation driver 6110 causes linear or axial motion of the intermediate articulation driver 6120 which causes the distal articulation link 6130 to articulate the elongated channel 1520. The shoulder 6119 serves to support the proximal articulation driver 6110 during operation and resists motion of the proximal articulation driver 6110 when encountering external forces that try to unintentionally de-articulate the end effector. The ACME thread arrangement 6116 establishes friction with the threaded passage segment 6122 in intermediate articulation driver 6120 so as to function as an articulation lock when the application of the rotary articulation motion to the proximal articulation driver 6110 has been discontinued. In at least one arrangement, the ACME thread arrangement 6116 may have a thread angle of less than 17° and be "self-locking". Other articulation lock arrangements may also be employed.

The devices disclosed herein can be designed to be disposed of after a single use, or they can be designed to be used multiple times. In either case, however, a device can be reconditioned for reuse after at least one use. Reconditioning can include any combination of the steps including, but not limited to, the disassembly of the device, followed by cleaning or replacement of particular pieces of the device, and subsequent reassembly of the device. In particular, a reconditioning facility and/or surgical team can disassemble a device and, after cleaning and/or replacing particular parts of the device, the device can be reassembled for subsequent use. Those skilled in the art will appreciate that reconditioning of a device can utilize a variety of techniques for disassembly, cleaning/replacement, and reassembly. Use of such techniques, and the resulting reconditioned device, are all within the scope of the present application.

The devices disclosed herein may be processed before surgery. First, a new or used instrument may be obtained and, when necessary, cleaned. The instrument may then be sterilized. In one sterilization technique, the instrument is placed in a closed and sealed container, such as a plastic or TYVEK bag. The container and instrument may then be placed in a field of radiation that can penetrate the container, such as gamma radiation, x-rays, and/or high-energy electrons. The radiation may kill bacteria on the instrument and in the container. The sterilized instrument may then be stored in the sterile container. The sealed container may keep the instrument sterile until it is opened in a medical facility. A device may also be sterilized using any other technique known in the art, including but not limited to beta radiation, gamma radiation, ethylene oxide, plasma peroxide, and/or steam.

## Claims

1. A surgical instrument (10), comprising:
a surgical end effector (1500);
an elongated shaft assembly (1000) defining a shaft axis and operably interfacing with said surgical end effector and comprising:
a first shaft actuator operably interfacing with a corresponding portion of said surgical end effector; and
a second shaft actuator operably interfacing with another corresponding portion of said surgical end effector and wherein said surgical instrument further comprises:
a first drive system configured to selectively apply axial actuation motions to said first shaft actuator; and
a clutch assembly (6300) movable between an engaged position wherein said clutch assembly converts said axial actuation motions of said first drive system to rotary actuation motions that are applied to said second shaft actuator and a disengaged position wherein said axial actuation motions of said first drive system are not applied to said second shaft actuator,
wherein said surgical end effector is configured to be articulated relative to said elongated shaft assembly by said second shaft actuator upon application of said rotary actuation motions to said second shaft actuator,
**characterized in that** said second shaft actuator comprises:
a proximal articulation driver (2110) interfacing with said clutch assembly to receive said rotary actuation motions therefrom; and
an intermediate articulation driver (2120) interfacing with said surgical end effector and threadably coupled to said proximal articulation driver such that rotation of said proximal articulation driver causes said intermediate articulation driver to axially move to apply articulation motions to said surgical end effector,
wherein said surgical instrument further comprises a distal articulation link (2130) coupled to said surgical end effector on one side of said shaft axis and attached to said intermediate articulation driver on another side of said shaft axis.

2. The surgical instrument of Claim 1, further comprising means for locking said second shaft actuator in position when said clutch assembly is in said disengaged position.

3. The surgical instrument of Claim 1, wherein said elongated shaft assembly further comprises a third shaft actuator configured to apply other axial actuation motions to said surgical end effector.

4. The surgical instrument of Claim 3, wherein said third shaft actuator is axially movable between an unactuated position and an actuated position wherein said other axial actuation motions are applied to said surgical end effector and wherein said surgical instrument further comprises a second drive system interfacing with said third shaft actuator to selectively move said third shaft actuator between said unactuated position and said actuated position.

5. The surgical instrument of Claim 4, wherein said third shaft actuator moves said clutch assembly from said engaged position to said disengaged position when said third shaft actuator is moved from said unactuated to said actuated position.

## Patentansprüche

1. Chirurgisches Instrument (10), umfassend:
einen chirurgischen Endeffektor (1500), umfassend;
eine längliche Wellenanordnung (1000), die eine Wellenachse definiert und mit dem chirurgischen Endeffektor in Wirkverbindung steht und umfassend:
ein erstes Wellenbetätigungselement, das mit einem entsprechenden Abschnitt des chirurgischen Endeffektors in Wirkverbindung steht; und
ein zweites Wellenbetätigungselement, das mit einem anderen entsprechenden Abschnitt des chirurgischen Endeffektors in Wirkverbindung steht, und wobei das chirurgische Instrument ferner umfasst:
ein erstes Antriebssystem, das konfiguriert ist, um wahlweise axiale Betätigungsbewegungen auf das erste Wellenbetätigungselement auszuüben; und
eine Kupplungsanordnung (6300), die zwischen einer eingerückten Position, wobei die Kupplungsanordnung die axialen Betätigungsbewegungen des ersten Antriebssystems in Drehbetätigungsbewegungen, die auf das zweite Wellenbetätigungselement ausgeübt werden, umwandelt, und einer ausgerückten Position, wobei die axialen Betätigungsbewegungen des ersten Antriebssystems nicht auf das zweite Wellenbetätigungselement ausgeübt werden, bewegbar ist,
wobei der chirurgische Endeffektor konfiguriert ist, um relativ zu der länglichen Wellenanordnung durch das zweite Wellenbetätigungselement bei Ausüben der Drehbetätigungsbewegungen auf das zweite Wellenbetätigungselement gelenkig bewegt zu werden,
**dadurch gekennzeichnet, dass** das zweite Wellenbetätigungselement umfasst:
einen proximalen Gelenktreiber (2110), der mit der Kupplungsanordnung in Verbindung steht, um davon die Drehbetätigungsbewegungen aufzunehmen; und
einen Zwischengelenktreiber (2120), der mit dem chirurgischen Endeffektor in Verbindung steht und mit dem proximalen Gelenktreiber gewindemäßig derart gekoppelt ist, dass Drehung des proximalen Gelenktreibers den Zwischengelenktreiber veranlasst, sich axial zu bewegen, um Gelenkbewegungen auf den chirurgischen Endeffektor auszuüben,
wobei das chirurgische Instrument ferner ein distales Gelenkglied (2130), das mit dem chirurgischen Endeffektor auf einer Seite der Wellenachse gekoppelt und an dem Zwischengelenktreiber auf einer anderen Seite der Wellenachse befestigt ist, umfasst.

2. Chirurgisches Instrument nach Anspruch 1, ferner umfassend Mittel zum Arretieren des zweiten Wellenbetätigungselements in Position, wenn die Kupplungsanordnung in der ausgerückten Position ist.

3. Chirurgisches Instrument nach Anspruch 1, wobei die längliche Wellenanordnung ferner ein drittes Wellenbetätigungselement, das konfiguriert ist, um andere axiale Betätigungsbewegungen auf den chirurgischen Endeffektor auszuüben, umfasst.

4. Chirurgisches Instrument nach Anspruch 3, wobei das dritte Wellenbetätigungselement zwischen einer unbetätigten Position und einer betätigten Position axial bewegbar ist, wobei die anderen axialen Betätigungsbewegungen auf den chirurgischen Endeffektor ausgeübt werden, und wobei das chirurgische Instrument ferner ein zweites Antriebssystem, das mit dem dritten Wellenbetätigungselement in Verbindung steht, um das dritte Wellenbetätigungselement zwischen der unbetätigten Position und der betätigten Position wahlweise zu bewegen, umfasst.

5. Chirurgisches Instrument nach Anspruch 4, wobei das dritte Wellenbetätigungselement die Kupplungsanordnung von der eingerückten Position in die ausgerückte Position bewegt, wenn das dritte Wellenbetätigungselement von der unbetätigten in die betätigte Position bewegt wird.

## Revendications

1. Instrument chirurgical (10), comprenant :
un effecteur terminal chirurgical (1500) ;
un ensemble tige allongée (1000) définissant un axe de tige et en communication fonctionnelle avec ledit effecteur terminal chirurgical et comprenant :
un premier actionneur de tige en communication fonctionnelle avec une partie correspondante dudit effecteur terminal chirurgical ; et
un deuxième actionneur de tige en communication fonctionnelle avec une autre partie correspondante dudit effecteur terminal chirurgical et dans lequel ledit instrument chirurgical comprend en outre :
un premier système d'entraînement conçu pour appliquer sélectivement des mouvements d'actionnement axiaux audit premier actionneur de tige ; et
un ensemble d'embrayage (6300) mobile entre une position en prise dans laquelle ledit ensemble d'embrayage convertit lesdits mouvements d'actionnement axiaux dudit premier système d'entraînement en mouvements d'actionnement rotatifs qui sont appliqués audit deuxième actionneur de tige, et une position hors prise dans laquelle lesdits mouvements d'actionnement axiaux dudit premier système d'entraînement ne sont pas appliqués audit deuxième actionneur de tige,
dans lequel ledit effecteur terminal chirurgical est conçu pour être articulé par rapport audit ensemble tige allongée par ledit deuxième actionneur de tige lors de l'application desdits mouvements d'actionnement rotatifs audit deuxième actionneur de tige,
**caractérisé en ce que** ledit deuxième actionneur de tige comprend :
un élément d'entraînement d'articulation proximal (2110) en communication avec ledit ensemble d'embrayage pour en recevoir lesdits mouvements d'actionnement rotatifs ; et
un élément d'entraînement d'articulation intermédiaire (2120) en communication avec ledit effecteur terminal chirurgical et accouplé par filetage audit élément d'entraînement d'articulation proximal de telle sorte qu'une rotation dudit élément d'entraînement d'articulation proximal amène ledit élément d'entraînement d'articulation intermédiaire à se déplacer axialement pour appliquer des mouvements d'articulation audit effecteur terminal chirurgical,
dans lequel ledit instrument chirurgical comprend en outre une liaison d'articulation distale (2130) accouplée audit effecteur terminal chirurgical d'un côté dudit axe de tige et fixée audit élément d'entraînement d'articulation intermédiaire d'un autre côté dudit axe de tige.

2. Instrument chirurgical selon la revendication 1, comprenant en outre un moyen permettant de verrouiller ledit deuxième actionneur de tige en position lorsque ledit ensemble d'embrayage est dans ladite position hors prise.

3. Instrument chirurgical selon la revendication 1, dans lequel ledit ensemble tige allongée comprend en outre un troisième actionneur de tige conçu pour appliquer d'autres mouvements d'actionnement axiaux audit effecteur terminal chirurgical.

4. Instrument chirurgical selon la revendication 3, dans lequel ledit troisième actionneur de tige est mobile axialement entre une position non actionnée et une position actionnée dans laquelle lesdits autres mouvements d'actionnement axiaux sont appliqués audit effecteur terminal chirurgical et dans lequel ledit instrument chirurgical comprend en outre un second système d'entraînement en communication avec ledit troisième actionneur de tige pour déplacer sélectivement ledit troisième actionneur de tige entre ladite position non actionnée et ladite position actionnée.

5. Instrument chirurgical selon la revendication 4, dans lequel ledit troisième actionneur de tige déplace ledit ensemble d'embrayage de ladite position en prise à ladite position hors prise lorsque ledit troisième actionneur de tige est déplacé de ladite position non actionnée à ladite position actionnée.
